# EUROPEAN PATENT APPLICATION

(11) **EP 3 799 722 A2**
(43) Date of publication of application: **07.04.2021**
(21) Application number: 20192478.4
(22) Date of filing: 30.06.2017
(51) Int. Cl.: A23K 10/18, A61K 31/732, A61K 31/733, A61K 35/741, A61K 35/745, A61K 35/747, A23K 20/163, A23K 50/75, A23L 33/135, A23L 33/21, A61P 1/12, A61K 31/715, A61K 45/06

(54) **COMPOSITION COMPRISING COMMENSAL PROBIOTIC AND PREBIOTIC**

(30) Priority: 30.06.2016 GB 201611486
(62) Divisional of application: 17748856.6
(71) Applicant: Dairy Crest Limited, Esher, Surrey KT10 9PN (GB)
(72) Inventor: CONNERTON, Ian, Loughborough, LE12 6LH (GB); CONNERTON, Phillippa, Loughborough, LE12 6LH (GB); FISH, Neville Marshall, Stockport, SK7 2PZ (GB); LAFONTAINE, Geraldine, Heanor, DE75 7SZ (GB); RICHARDS, Phillip, Nottingham, NG2 5JT (GB)
(74) Representative: Wilson Gunn

(57) **Abstract**

A composition for the treatment and/or nutrition of poultry such as broiler chickens is disclosed as comprising (i) one more probiotics which are commensal selected from one or more of ( ), ( ), ( ), ( ) or *;* and (ii) a prebiotic material.

## Description

The present invention relates to compositions for use in the treatment and/or nutrition of poultry, such as broiler chickens (*Gallus gallus domesticus*).

Broiler chickens are the most widely farmed animals. Around 50 billion chickens are reared each year for global consumption. Chicken farming on an industrial scale presents significant challenges both of a practical and animal welfare nature. Birds which are densely stocked, even in a free-range environment, will be apt to transmit bacterial disease. Enteric bacterial infections such as *Campylobacter jejuni* are both prevalent and undesirable in broilers. One of the major indications for the use of antibiotics in broilers is enteric disease (Journal of Antimicrobial Chemotherapy, Vol 61, Issue 4, Pp947-952).

Studies have demonstrated that certain commensal bacteria present in the microbiota of poultry such as broilers can have a beneficial effect upon their rearing, by improving their gut health and thereby their performance in terms of feed conversion ratio (FCR) and rate of weight gain (see for example, "Microbiota of the chicken gastrointestinal tract: influence on health, productivity and disease", Stanley et al Appl. Microbiol. Biotechnol. (2014) 98:4301-4310; and Stanley D, Hughes RJ, Geier MS and Moore RJ(2016) Bacteria within the gastrointestinal tract microbiota correlated with improved growth and feed conversion: Challenges presented for the identification of performance enhancing probiotic bacteria. Front. Microbiol. 7:187. doi:10.3389/fmicb.2016.00187).

Reference herein to the bacteria as being commensal refers to their presence within the gastrointestinal tract of the majority of the broiler populations. However, it is the case that because of the environment, diet, broiler stock or other factors that either a particular broiler population or, for whatever reason, a proportion of broilers within a population, have an altered microbiota or lack one or more of those bacteria.

It would therefore be desirable to identify specific probiotics for poultry such as broilers comprising one or more such bacteria. The use of such a probiotic will, therefore, result in an improvement in the profile of commensal bacteria within a broiler chicken, since it will then include one or more of these bacteria shown to be beneficial to rearing, which have a beneficial effect upon broiler health and performance.

Therefore, according to the present invention, there is provided a composition comprising:
(i) a probiotic selected from one or more of the bacteria *Bifidobacterium animalis, Collinsella tanakaei, Lactobacillus reuteri, Anaerostipes, Lactobacillus crispatus, Pediococcus acidilactici, Lactobacillus pontis, Faecalibacterium prausnitzii, Coprococcus catus, Roseburia intestinalis, Anaerostipes butyraticus, Butyricicoccus, Lactobacillus johnsonii, and Ruminococcus sp.* ; and
(ii) a prebiotic material.

The composition of the invention may comprise the specific probiotic bacterial strain *Lactobacillus crispatus* DC21.1 (NCIMB 42771), deposited on 23 June 2017 at NCIMB Limited, Ferguson Building, Craibstone Estate, Bucksburn, Aberdeen, AB21 9YA,. United Kingdom.

The composition of the invention may comprise the specific probiotic bacterial strain *Lactobacillus johnsonii* DC22.2 (NCIMB 42772), deposited on 23 June 2017 at NCIMB Limited, Ferguson Building, Craibstone Estate, Bucksburn, Aberdeen, AB21 9YA,. United Kingdom.

The composition of the invention may comprise the specific probiotic bacterial strain *Lactobacillus reuteri* DC1B4 (NCIMB 42773), deposited on 23 June 2017 at NCIMB Limited, Ferguson Building, Craibstone Estate, Bucksburn, Aberdeen, AB21 9YA,. United Kingdom.

The composition of the invention may comprise the specific probiotic bacterial strain *Ruminococcus* sp. DC3A4 (NCIMB 42774), deposited on 23 June 2017 at NCIMB Limited, Ferguson Building, Craibstone Estate, Bucksburn, Aberdeen, AB21 9YA,. United Kingdom.

The probiotic bacteria used in the invention are typically commensal bacteria.

An example of the performance objectives for broiler chickens can be found in *Aviagen Ross 308 Broiler Performance Objectives 2014* documentation.

An example of the nutrition specifications for broiler chickens can be found in *Aviagen Ross 308 Broiler Nutrition Specifications 2014* documentation.

Prebiotic materials are defined by the US Food and Drug Administration as being non-digestible food ingredients that beneficially affect the host by selectively stimulating the growth and/or activity of one or a limited number of bacteria in the colon.

The definition provided by the US Food and Drug Administration has been reviewed and modified based on three criteria:
(a) resistance to gastric acidity, hydrolysis by mammalian enzymes and gastrointestinal absorption;
(b) fermentation by intestinal microflora;
(c) selective stimulation of the growth and/or activity of intestinal bacteria associated with health and well-being;

In view of this, prebiotic materials are defined by Gibson *et al.* (2004) *(*Gibson, G. R., Probert, H. M., Loo, J. V., Rastall, R. A., and Roberfroid, M. B. (2004) "Dietary modulation of the human colonic microbiota: updating the concept of prebiotics" Nutrition Research Reviews, 17(2) 259-275*)* as being a selectively fermented ingredient that allows specific changes, both in the composition and/or activity in the gastrointestinal microflora that confers benefits upon host well-being and health.

Examples of prebiotics are inulin, fructo-oligosaccharides (also known as oligofructose) which is a partial hydrolysate of inulin, galacto-oligosaccharides (GOS) (also known as transgalacto-oligosaccharides), lactulose, lactosucrose, isomalto-oligosaccharides, xylo-oligosaccharides, arabinoxylo-oligosaccharides, gluco-oligosaccharides, mannan oligosaccharides (MOS), soyabean oligosaccharides, and pectic oligosaccharides.

### Prebiotic definitions

The following definitions provided by Gibson *et al.* (2004) are accepted as standard definitions of the above-mentioned examples of prebiotics:

### Inulin and fructo-oligosaccharides

Inulin, or the hydrolysed fructo-oligosaccharides, are described as either an:
- α-D-glucopyranosyl-[β-D-fructofuranosyl]ₙ₋₁-β-D-fructofuranoside (GFₙ); or a
- β-D-fructopyranosyl-[β-D-fructofuranosyl]ₙ₋₁-β-D-fructofuranoside.

The fructosyl-glucose linkage is always β(2←1) as in sucrose, but the fructosyl-fructose linkages are β(1←2).

There are a number of sources of inulin. A major source of inulin is chicory. Chicory inulin is composed of a mixture of oligomers and polymers in which the degree of polymerisation (DP) varies from 2 - 60 with an average DP ≈ 12.

Fructo-oligosaccharides (oligofructose) are formed by the partial (enzyme catalysed or chemical) hydrolysis of inulin giving a mixture of both α-D-glucopyranosyl-[β-D-fructofuranosyl]ₙ₋₁-β-D-fructofuranoside (GFₙ) and β-D-fructopyranosyl-[β-D-fructofuranosyl]ₙ₋₁-β-D-fructofuranoside molecules with a DP of 2 - 7.

### Galacto-oligosaccharides (GOS)

GOS are a mixture of oligosaccharides formed by the enzyme (β-galactosidase) catalysed transglycosylation of lactose and subsequent galacto-oligosaccharides. The oligosaccharides are often considered to be of the form (gal)ₙ-glc with DP = 2 - 8 and β(1→6), β(1→4) and β(1→4) mixed linkages; however, galactans with the same linkages can be present. The product mixtures depend upon the enzymes used and the reaction conditions.

GOS (galacto-oligosaccharide) is sold by Dairy Crest Ltd under the trade name Nutrabiotic® GOS for animal feed applications.

### Lactulose

Lactulose is manufactured by the isomerisation (often chemical isomerisation) of lactose to generate the disaccharide galactosyl-β(1→4)-fructose.

### Lactosucrose

Lactosucrose is_produced from a mixture of lactose and sucrose in an enzyme (for example β-fructofuranosidase) catalysed transglycosylation reaction. The fructosyl residue is transferred from sucrose to the C 1 position of the glucose moiety in the lactose, producing a non-reducing oligosaccharide.

### Isomalto-oligosaccharides

Isomalto-oligosaccharides are_manufactured from malto-oligosaccharides, or maltose (both of which are produced from starch by the combined reactions catalysed by α-amylase and pullulanase, or β-amylase and pullulanase). The malto-oligosaccharides and maltose are converted into α(1→6)-linked isomalto-oligosaccharides by enzyme (a-glucosidase or transglucosidase) catalysed transglycosylation reactions.

### Xylo-oligosaccharides and arabinoxylo-oligosaccharides

Xylo-oligosaccharides and arabinoxylo-oligosaccharides are_made from wood or cereal non-starch materials (corn cobs, wheat bran etc.). Depending upon various xylan sources used, and the method of production, the structures vary in degree of polymerization, monomeric units, and types of linkages. Generally, xylo-oligosaccharides are mixtures of oligosaccharides formed from xylose residues, typically DP = 2 - 10, linked through β(1→4)-linkages. Xylan is usually found in combination with other side groups such as α-D-glucopyranosyl uronic acid or its 4-O-methyl derivative, acetyl groups or arabinofuranosyl (giving arabinoxylo-oligosaccharides) residues.

Xylo-oligosaccharides and arabinoxylo-oligosaccharides are produced by chemical methods, enzyme catalysed hydrolysis (e.g. the hydrolysis of arabinoxylans catalysed by combinations of endo-1,4-β-xylanases, β-xylosidases, arabinofuranosidases and feruloyl esterases) or a combination of chemical and enzyme catalysed treatments.

### Gluco-oligosaccharides

Gluco-oligosaccharides are often referred to as α-GOS. These are mixed α-gluco-oligosaccharides produced in reactions catalysed by dextran sucrase in fermentation processes (fermentation of *Leuconostoc mesenteroides*) or in the enzyme catalysed transglycosylation reactions involving sucrose in the presence of maltose. This gives oligosaccharides with a range of α-linkages (e.g. glucosyl-α(1→2)-glucosyl-α(1→6)-glucosyl-α(1→4)-glucose).

### Mannan oligosaccharides (MOS)

Mannan oligosaccharides are normally obtained from the cell walls of the yeast *Saccharomyces cerevisiae.* and presented as products of different levels of purity. In the yeast cell wall, mannan oligosaccharides are present as:
- complex molecules that are linked to the cell wall proteins as -O and -N glycosyl groups;
- α-D-mannans made up of an α-(1,6)-D-mannose backbone to which are linked α-(1,2)- and α-(1,3)- D-mannose branches (1 - 5 mannosyl groups long).

### Soyabean oligosaccharides

Soyabean oligosaccharides are α-galactosyl sucrose derivatives (e.g. raffinose, stachyose, verbascose). They are isolated from soya beans and concentrated for the final product formulation.

### Pectic oligosaccharides

Pectic oligosaccharides (POS) are obtained by pectin depolymerization by either enzyme (pectin hydrolases and lyases) catalysed reactions or acid (typically) hydrolysis. Given that pectins are complex ramified heteropolymers made up of:
- a smooth region of linear backbone of α(1→4)-linked D-galacturonic acid units which can be randomly acetylated and/or methylated);
- hairy regions of rhamnogalacturonan type I and rhamnogalacturonan type II;
the structural diversity of the pectic oligosaccharides from pectin hydrolysis is high.

The prebiotic materials useful in the invention may be naturally or non-naturally occurring. The probiotics are responsive to prebiotics, with the populations of the probiotics increasing due to the presence of the prebiotic material, and the presence of the prebiotic material correlates with improved broiler performance, including weight gain during rearing.

The one or more bacteria are typically selected from the more specific bacterial strains, as identified as nearest cultural examples: *Bifidobacterium animalis* subsp. *lactis* str. V9, *Collinsella tanakaei* str. YIT 12064, *Lactobacillus reuteri* str. BCS136, *Anaerostipes* sp. str. 35-7, *Lactobacillus crispatus* str. ST1, *Lactobacillus crispatus* str. DC21, *Lactobacillus crispatus* str. DC21.1 (NCIMB 42771), *Lactobacillus johnsonii* str. DC22.2 (NCIMB 42772), *Lactobacillus reuteri* str. DC1B4 (NCIMB 42773), and *Ruminococcus sp.* str. DC3A4 (NCIMB 42774).

The probiotic bacteria used in the invention were identified as being up-regulated in a broiler trial treatment that contained galacto-oligosaccharides (GOS) in the feed, compared to a control feed.

The one or more bacteria may be selected from their nearest (based on sequence) equivalents. Identification of the bacteria included in the composition of the invention is based on Operational Taxonomic Units (OTUs) identified from 16S rDNA sequences from the V4 region of the microbiome. Specifically, 16S rRNA gene sequences were aligned against a reference alignment based on the SILVA rRNA database and clustered into OTUs with an average neighbor clustering algorithm. The nearest 16S rRNA gene sequence identities to the OTUs are reported on the basis of BLASTn searches if data matches are from type cultures with a BLAST identity ≥99%. The laboratory and bioinformatic techniques used to identify the bacteria included in the composition of the invention is described as follows:

### Histology

Samples of ileum for histological assessment were examined from birds from each relevant treatment. The fixed tissue samples were dehydrated through a series of alcohol solutions, cleared in xylene, and finally embedded in paraffin wax (Microtechnical Services Ltd, Exeter, UK). Sections (3 to 5 µm thick) were prepared and stained with modified hematoxylin and eosin (H&E) using standard protocols. After staining, the slides were scanned by NanoZoomer Digital Pathology System (Hamamatsu, Welwyn Garden City, UK). Measurements of villus height and crypt depth were made using the NanoZoomer Digital Pathology Image Program (Hamamatsu) of 10 well-oriented villi scanned at 40 X magnification. Villus height was measured from the tip of the villus to the crypt opening and the associate crypt depth was measured from the base of the crypt to the level of the crypt opening. The ratio of villus height to relative crypt depth (V:C ratio) was calculated from these measurements.

### RNA Isolation and RT-qPCR of the Cytokines and Chemokines

RNA was isolated from cecal and ileal tissue biopsies using NucleoSpin RNA isolation kit (Macherey-Nagel, GmbH & co. KG, Düran DE) according to the manufacturer's protocol with the following modifications. Tissue samples were homogenized in Lysis buffer with 2.8 mm ceramic beads (MO BIO Laboratories Inc., Carlsbad, USA) using TissueLyser II (Qiagen, Hilden, DE) prior to subsequent purification as described in the protocol. RNA was eluted in DEPC treated water (Ambion ThermoFisher Scientific, UK) and stored at -80°C. RNA quality and concentration were assessed using Nanodrop ND-1000 Spectrophotometer (Labtech International Ltd, Uckfield, UK). The ratio 260/280 nm was in the range of 1.79 to 2.17 with the mean of 2.12 ±0.01 for all RNA samples used.

Reverse Transcription was performed with 1µg of RNA using SuperScript II (Invitrogen Life Technologies, Carlsbad, USA.) and random hexamers (Untergasser's Lab 2008 accessed online 16/12/2016; URL http://www.untergasser.de/lab/protocols/cdna_synthesis_superscript_ii_v1_0.htm). Quantitative PCR reaction was performed with cDNA template derived from 4 ng of total RNA in triplicate using SYBR Green Master mix (Applied Biosystems, ThermoFisher Scientific). Cytokines and chemokines fold change were calculated using the "comparative Cycle threshold (Ct) method" established by the manufacturer as described by Livak, K.J., and Schmittgen, T.D. (2001). Analysis of Relative Gene Expression Data Using Real-Time Quantitative PCR and the 2-ΔΔCT Method. Methods 24, 402-408.. The average of the triplicate Ct values was used for analysis and the target genes Ct values were normalized to those of the housekeeping gene encoding Glyceraldehyde 3-phosphate dehydrogenase (GAPDH). The RNA level of expression was determined by qPCR using the Roche Diagnostics LightCycler 480 (Hoffmann La Roche AG, CH). The primers used for qPCR of GAPDH, IFN-γ, IL-1β, IL-4, IL-6, IL-10, IL-17A, IL-17F, CXCLi1 and CXCLi2 are presented in Table 6.

### DNA Extraction and PCR Amplification of 16S rRNA Gene Sequences and Microbiota Diversity Analysis

Bacterial DNA was isolated from 0.25 g cecal content using the PowerSoil DNA Isolation Kit (MO Bio Laboratories) according to the manufacturer's instructions. Using the isolated DNA as a template the V4 region of the bacterial 16S rRNA gene was PCR amplified using primers 515f(5' GTGCCAGCMGCCGCGGTAA 3') and 806r (5' GGACTACHVGGGTWTCTAAT 3') as described by Caporaso, J.G., Lauber, C.L., Walters, W.A., Berg-Lyons, D., Lozupone, C.A., Turnbaugh, P.J., et al. (2011). Global patterns of 16S rRNA diversity at a depth of millions of sequences per sample. Proc. Natl. Acad. Sci. U S A. 108 Suppl 1, 4516-4522. doi: 10.1073/pnas.1000080107.

Amplicons were then sequenced on the Illumina MiSeq platform using 2 x 250 bp cycles. Prior to metagenomic analysis sequence reads with a quality score mean below 30 were removed using Prinseq (Schmieder, R., and Edwards, R. (2011) Quality control and preprocessing of metagenomic datasets. Bioinformatics 27, 863-864. doi: 10.1093/bioinformatics/btr026.). The 16S rRNA sequence analysis was performed using Mothur v. 1.37.4 (Schloss, P.D., Westcott, S.L., Ryabin, T., Hall, J.R., Hartmann, M., Hollister, E.B., et al. (2009). Introducing mothur: open-source, platform-independent, community-supported software for describing and comparing microbial communities. Appl. Environ. Microbiol. 75, 7537-7541. doi: 10.1128/AEM.01541-09.). Analysis was performed as according to the MiSeq SOP (accessed online 08/12/2016; Kozich, J.J., Westcott, S.L., Baxter, N.T., Highlander, S.K., and Schloss, P.D. (2013). Development of a dual-index sequencing strategy and curation pipeline for analyzing amplicon sequence data on the MiSeq Illumina sequencing platform. Appl. Environ. Microbiol. 79, 5112-5120.) with the exception that the screen.seqs command used a maxlength option value similar to that of the 97.5 percentile length. The 16S rRNA gene sequences were aligned against a reference alignment based on the SILVA rRNA database (Pruesse, E., Quast, C., Knittel, K., Fuchs, B.M., Ludwig, W.G., Peplies, J., et al. (2007). SILVA: a comprehensive online resource for quality checked and aligned ribosomal RNA sequence data compatible with ARB. Nucl. Acids Res. 35, 7188-7196 doi: 10.1093/nar/gkm864) for use in Mothur (available at: https://www.mothur.org/wiki/Silva reference files), and clustered into operational taxonomic units (OTUs) with an average neighbor clustering algorithm. The nearest 16S rRNA gene sequence identities to the OTUs are reported on the basis of BLASTn searches if data matches are from type cultures with a BLAST identity ≥99%. If not, the consensus taxonomy of the OTUs is reported as generated using the classify.otu command in Mothur with reference data from the Ribosomal Database Project (version 14) (Cole, J. R., Wang, Q., Fish, J.A., Chai, B., McGarrell, D. M., Sun, Y., et al. (2014). Ribosomal Database Project: data and tools for high throughput rRNA analysis. Nucl. Acids Res. 42(Database issue), D633-D642.; Wang, Q., Garrity, G. M., Tiedje, J. M., and Cole, J.R. (2007). Naive Bayesian classifier for rapid assignment of rRNA sequences into the new bacterial taxonomy. Appl Environ Microbiol. 73, 5261-5267. doi: 10.1128/AEM.00062-07) adapted for use in mothur (available at: https://www.mothur.org/wiki/RDP reference files).

### Data Analysis

ANOVA followed by Tukey's multiple comparisons test and Kruskal-Wallis test followed by Dunn's multiple comparisons test was performed using GraphPad Prism version 7.00 for Windows (GraphPad Software, La Jolla, USA, www.qraphpad.com). Metastats were implemented within Mothur (White, J.R., Nagarajan, N., and Pop, M. (2009). Statistical methods for detecting differentially abundant features in clinical metagenomic samples. PLoS Comput. Biol. 5:e1000352. doi: 10.1371/journal.pcbi.1000352). Data processing and ordination were performed using R project (R Development Core Team, 2008. R: A language and environment for statistical computing. R Foundation for Statistical Computing, Vienna, Austria. ISBN 3-900051-07-0; URL http://www.R-project.org). Heatmaps were plotted using the heatmap.2 function of R package gplots (Warnes, G.R., Bolker, B., Bonebakker, L., Gentleman, R., Huber, W., Liaw, A., et al. (2016). gplots: Various R Programming Tools for Plotting Data. R package version 3.0.1. https://CRAN.R-project.org/package=gplots).

### Ethics Statement

Studies were carried out under license and in accordance with UK Animals (Scientific Procedures) Act 1986. All procedures were approved by the Local Ethics Committee of the University of Nottingham.

The most preferred one or more bacteria are selected from the specific bacterial strains *Lactobacillus crispatus* str. DC21.1 (NCIMB 42771), *Lactobacillus johnsonii* str. DC22.2 (NCIMB 42772), *Lactobacillus reuteri* str. DC1B4 (NCIMB 42773), and *Ruminococcus* sp. str. DC3A4 (NCIMB 42774).

Preferable compositions of the present invention are *Lactobacillus crispatus* str. DC21.1 (NCIMB 42771) with a galacto-oligosaccharide, such as Nutrabiotic® GOS, *Lactobacillus johnsonii* str. DC22.2 (NCIMB 42772) with a galacto-oligosaccharide, such as Nutrabiotic® GOS, *Lactobacillus reuteri* str. DC1B4 (NCIMB 42773) with a galacto-oligosaccharide, such as Nutrabiotic® GOS, and *Ruminococcus* sp. str. DC3A4 (NCIMB 42774) with a galacto-oligosaccharide, such as Nutrabiotic® GOS.

The strains *Lactobacillus crispatus* str. DC21.1 (NCIMB 42771), *Lactobacillus johnsonii* str. DC22.2 (NCIMB 42772), *Lactobacillus reuteri* str. DC1B4 (NCIMB 42773), and *Ruminococcus* sp. str. DC3A4 (NCIMB 42774), are all commensal to Ross 308 broilers grown on a standard wheat-based feed that also contains Nutrabiotic® GOS (galacto-oligosaccharide) and produced in the poultry facility, University of Nottingham, Sutton Bonington campus, and were isolated from digesta taken from the caecum.

The specific bacterial strains, as well as the bacteria (not sequenced), have been identified from the microbiome of the same.
It is reported in Stanley, D., Hughes, R. J., and Moore, R. J. (2014) "Microbiota of the chicken gastrointestinal tract: influence on health, productivity and disease" Applied Microbiology and Biotechnology, 98 4301-4310 and Stanley D, Hughes RJ, Geier MS and Moore RJ(2016) Bacteria within the gastrointestinal tract microbiota correlated with improved growth and feed conversion: Challenges presented for the identification of performance enhancing probiotic bacteria. Frontiers in Microbiology, 7:187. doi:10.3389/fmicb.2016.00187 that the bacteria and specific bacterial strains are associated with good outcomes, and/or are associated with the microbiota of broilers that display high performance.

According to one embodiment of the invention, the composition may comprise two or more probiotics. For example, a first probiotic preparation may be taken from a group comprising specific facultative anaerobic commensal bacteria, for example *Lactobacillus* spp. and *Bifidobacterium* spp., which produce acetate and lactate when acting on a prebiotic, and a second probiotic preparation may be taken from a group comprising specific strictly anaerobic commensal bacteria which produce butyrate "feeding" on the acetate and lactate produced by the probiotic preparation of the first group. A 'probiotic preparation' is considered to comprise one or more probiotic bacteria taken from the respective facultative anaerobic or strictly anaerobic group.

According to another embodiment of the invention, the composition may comprise the two or more probiotics in combination with only one prebiotic material. An example of a potential combination of a composition according to this embodiment may be a first probiotic, for example *Lactobacillus* spp. or *Bifidobacterium* spp., taken from a group comprising specific facultative anaerobic commensal bacteria which produce acetate and lactate when acting on the prebiotic, and a second probiotic taken from a group comprising specific strictly anaerobic commensal bacteria which produce butyrate "feeding" on the acetate and lactate produced by the first probiotic, in combination with a prebiotic, for example, Nutrabiotic® GOS.

The bacteria may comprise facultative anaerobic bacteria or strictly anaerobic bacteria

According to another embodiment of the invention, the composition may comprise facultative anaerobic bacteria in combination with a prebiotic. The combination may create acetate and lactate.

According to another embodiment of the invention, the composition may comprise strictly anaerobic bacteria in combination with acetate and lactate. The combination may create organic acids. The organic acids may be, for example, butyrate.

The prebiotic material used in the composition of the invention is typically substantially indigestible in the gastrointestinal system of a chicken.

Another aspect of the present invention was to identify specific probiotics which respond favourably to the use of polymeric saccharide, such as an oligosaccharide sugar, as a prebiotic material; and whose populations with the broiler gastrointestinal tract can, therefore, be increased by the use of such prebiotics. Therefore, the prebiotic material is typically a polymeric saccharide, such as an oligosaccharide.

The oligosaccharide used in the composition of the invention may be selected from one or more of fructooligosaccharide (also known as oligofructose) which is a partial hydrolysate of inulin, mannanoligosaccharide (MOS), galactooligosaccharide (GOS), xylooligosaccharide, arabinoxylanoligosaccharide, soyoligosaccharide, lactulose, lactosucrose, isomalto-oligosaccharides, gluco-oligosaccharides, pectic oligosaccharides, and inulin. Typically, the oligosaccharide is a galactooligosaccharide.

Galactooligosaccharides (GOS) have the general form (galactosyl)ₙ-lactose and typically range in size from trisaccharides to octasaccharides. Structural complexity is introduced by the different intermolecular bonds. Products said to comprise GOS therefore typically contain a mixture of galactooligosaccharides, lactose, glucose and galactose, and the term GOS is used herein in a manner intended to encompass such products.

GOS (galacto-oligosaccharide) is sold by Dairy Crest under the trade name Nutrabiotic® GOS for animal feed.

Typically, Nutrabiotic® GOS L is used as the prebiotic in the composition of the present invention. Nutrabiotic® GOS L complies with UK and EU Regulations and recommended purity specifications, including heavy metals, for feed and food ingredients. An analysis of Nutrabiotic® GOS L is provided in Table 21.

The recommended inclusion, or dose, rate of Nutrabiotic® GOS in animal feed diets depends on a number of factors. For example:
- the animal (e.g. broiler (chicken for fattening) or piglet);
- life cycle and the feeding regime (e.g. the different feeds being used and the duration of their use; use, and commencement of use, of a creep (pre-starter) feed;
   age of piglets at weaning etc.);
- formulation of the Nutrabiotic® GOS product and, to a lesser extent, the batch of the Nutrabiotic® GOS product being used.

The data presented in Table 22 are recommendations based on typical feeding regimes and ones that have been used in both research and commercial trials. They can be modified as required.

The data presented in Table 24 provides an estimate of the metabolizable energy values of Nutrabiotic® GOS L in broilers and piglets.

Nutrabiotic® GOS contains no significant quantities of protein or fat, or vitamins, minerals etc. as shown in Table 21. Nutrabiotic® GOS contains a range of carbohydrates that
are either digested as sugars, or fermented as soluble fibre. In the context of energy value for animal feed applications and specific animals, the definition of what is considered fibre is
complicated as an appreciable number of disaccharides present in Nutrabiotic® GOS L are fermented. Moreover the proportion of disaccharides that are fermented will differ depending on the animal (e.g. piglets compared to poultry).

Starter, grower and finisher refer to the diets at the different stages of the broiler production cycle. The diets correspond to the following periods (day 0 is defined as the day the broiler chicks are "placed" in the poultry shed, although at day 0 the broiler chicks are usually 1 day old):

| | |
|---|---|
| • 0 - 10 days | Starter feed (sieved crumb, but can alternatively be in the form of a mash feed) |
| • 11-24 days | Grower feed (pellets 3 mm diam.) |
| • 25-35 days | Finisher feed (pellets 3 mm diam.) |

The feeds, after the mixing of all the raw materials are pelleted (after steam injection and treatment) are extruded through a defined die to typically give a 3 mm pellet, that is the final broiler feed.

The pelleting process may follow typical methods known to a person skilled in the art.

Suitable feed and pellet size may be known to a person skilled in the art.

Crumb refers to a crumbed (broken into crumb) pelleted feed - typically to give smaller feed pieces that the broiler chicks can manage. A mash feed (a feed mixture that has not been pelleted) may be used instead of a crumb feed for the started feed.

The production cycle in this example is 35 days, which is reasonably common for experiments involving male (we only use the faster growing males to decrease the statistical variation in experimental systems) Ross 308 birds. Poultry cycles are more complex with birds being "harvested" at 35 - 42 days to get different weight ranges for commercial purposes.

Typically, the production cycle is 35 days, which is reasonably common for experiments involving male Aviagen Ross 308 birds, as typically used in the present invention.

The composition of the invention typically includes an amount of between about 10⁴ colony forming units (cfu) to 10¹² cfu, typically between about 10⁵ cfu to 10¹⁰ cfu, more typically between about 10⁶ cfu to 10⁸cfu, and most typically 10⁷ cfu. CFU is essentially the number of live bacteria added at day 9 of a trial. Preferably, the addition of CFU should not preclude the probiotic being added at different times, or continuously, as part of the feed, in a commercial operation.

The composition of the invention includes a prebiotic, typically Nutrabiotic® GOS.

Typically, a starter feed includes an amount of prebiotic, for example Nutrabiotic® GOS, between about 55% to 95% (w/w) solids concentration syrup, typically between about 65% to 85% (w/w) solids concentration syrup, more typically between about 70% to 80% (w/w) solids concentration syrup, and most typically about 75% (w/w) solids concentration syrup.

Typically, in the starter feed the prebiotic, for example Nutrabiotic® GOS, is added at a dose rate between about 0.50% to 5.00% (w/w complete starter feed), typically between about 1.50% to 3.50% (w/w complete starter feed), more typically between about 2.00% to 3.00%, even more typically between about 2.20% to 2.60% (w/w complete starter feed), even more typically between about 2.40% to 2.50%, and most typically about 2.47% (w/w complete starter feed).

Typically, a grower feed includes an amount of prebiotic, for example Nutrabiotic® GOS, between about 55% to 95% (w/w) solids concentration syrup, typically between about 65% to 85% (w/w) solids concentration syrup, more typically between about 70% to 80% (w/w) solids concentration syrup, and most typically about 75% (w/w) solids concentration syrup.

Typically, in the grower feed the prebiotic, for example Nutrabiotic® GOS, is added at a dose rate between about 0.20% to 5.00% (w/w complete grower feed), typically between about 0.60% to 3.50% (w/w complete grower feed), more typically between about 0.90% to 2.80%, even more typically between about 1.10% to 2.00% (w/w complete grower feed), even more typically between about 1.15% to 1.60%, even more typically between about 1.20% to 1.40%, and most typically about 1.24% (w/w complete grower feed).

Typically, the prebiotic, for example, Nutrabiotic® GOS, is not added to the finisher feed.

In a typical trial experiment, the addition of the bacteria is typically made in 0.10ml of MRD (Maximum Recovery Diluent), giving 10⁷ cfu (colony forming units) or viable cells, by cloacal gavage.

A further aspect of the present invention relates to a composition as defined hereinabove for the treatment and/or nutrition of poultry, such as broiler chickens, to which at least one of the probiotics responds to produce an increase in population.

The composition of the invention may also further comprise a nutrient food source. The nutrient food source may contain a source of protein, starch, amino acids, fat, or a combination of any two or more thereof. The nutrient food source may also contain one or more food additives which can be found in poultry feed, such as, but not limited to, vaccines, antibiotics, and coccidiostats, or a combination thereof. The antibiotics may be those used in treatment or as growth promoters.

The composition of the invention, containing the probiotic bacteria which are responsive to the prebiotics, and whose presence correlates with improved broiler performance, is able to impart benefits to the development of the poultry compared with poultry which is not exposed to the composition, such as an increased rate of growth, and/or a higher final weight, and/or a larger ratio of kilograms of feed required per kilogram of growth of the poultry.

The inventors have been able to show that gastrointestinal populations of the probiotic bacteria respond to the administration of prebiotics, such as oligosaccharides; and that increases in populations of one or more of the probiotic bacteria correlate to improved weight gain within broilers.

Also provided by the present invention is a composition for use in the treatment of enteric bacterial disease in poultry, the composition comprising:
(i) a probiotic selected from one or more of the bacteria *Bifidobacterium animalis, Collinsella tanakaei, Lactobacillus reuteri, Anaerostipes, Lactobacillus crispatus, Pediococcus acidilactici, Lactobacillus pontis, Faecalibacterium prausnitzii, Coprococcus catus, Roseburia intestinalis, Anaerostipes butyraticus, Butyricicoccus, Lactobacillus johnsonii, and Ruminococcus* sp.; and
(ii) a prebiotic material.

The definitions and embodiments defined above for the composition of the invention also apply to the composition for use in the treatment of enteric bacterial disease in poultry.

The enteric bacterial disease is infection by one or more of the following: *Clostridium perfringens, Salmonella* spp, pathogenic and toxigenic *Escherichia coli* (EPEC and ETEC).

The composition of the invention may be administered in any suitable manner, including, but not limited to, orally (via feed, which may need to be encapsulated in order to protect the probiotic from the acidic environment in a chicken's stomach), via intracloacal delivery (Arsi, Donoghue, Woo-Ming, Blore and Donoghue: Intracloacal Inoculation, an Effective Screening Method for Determining the Efficacy of Probiotic Bacterial Isolates against Campylobacter Colonisation in Broiler Chickens, Journal of Food Protection, Vol 78, No. 1 2015, Pages 209-213), or via a spray, such as onto chicks so they consume the composition by licking their feathers.

A further aspect of the present invention is a composition for the treatment and/or nutrition of poultry, such as a broiler chicken, comprising one or more specific probiotics and a prebiotic material which produce organic acids in the gastrointestinal tract, which impart benefits to the health of the broiler chickens.

All of the probiotics listed hereinabove are able to act in an strictly anaerobic manner, while some are also able to act in a facultative anaerobic manner.

It is those (e.g. *Lactobacillus* spp. and *Bifidobacterium* spp.) which act in a facultative anaerobic manner which produce organic acids, such as acetic and lactic acids, in the gastrointestinal tract such as acetic and lactic acids, when fermenting the prebiotic. The probiotics which are strict anaerobes, produce butyrate and other organic acids when supplied with a prebiotic and the acetate and lactate. These probiotics include, for example, *Coprococcus catus, Roseburia intestinalis,* and *Anaerostipes butyraticus, Ruminococcus sp., Butyricicoccus, and Faecalibacterium prausnitzii.*

These bacteria are known to feed upon fibre in the gastrointestinal tract of a broiler chicken. That feeding process generates the organic acids which are beneficial in at least two ways. Firstly, they reduce the pH within the tract which, generally speaking, tends to assist the growth of beneficial gut flora whilst simultaneously inhibiting the growth of more harmful flora. Secondly, the acids are directly beneficial *per se* as nutrients to the broiler and so the presence of one or more of these bacteria produces useable sources of energy.

The probiotics used in the invention serve the additional benefit of reducing populations of harmful gut flora. Examples of such harmful flora are *Clostridium perfringens* which is known to cause necrotic enteritis, and *Salmonella* whose presence is extremely harmful to humans and so desirably eliminated from broilers.

Although one or more of the compositions set out above can be used to treat, for example, the presence of undesirable gut flora in broiler chickens, they may advantageously also be used in feed compositions for prophylactic purposes.

The invention will now be described further by way of example with reference to the following examples, which are intended to be illustrative only and in no way limiting upon the scope of the invention.

### Examples

An example of a trial experiment using the composition of the invention included the following:
- prebiotic (Nutrabiotic® GOS) at the following dose rates:
   ▪ starter feed: Nutrabiotic® GOS a 75 %(w/w) solids concentration syrup added at a dose rate of 2.47 %(w/w complete starter feed)
   ∘ grower feed: Nutrabiotic® GOS a 75 %(w/w) solids concentration syrup added at a dose rate of 1.235 %(w/w complete grower feed)
   ∘ finisher feed: Nutrabiotic® GOS is not added to the finisher feed;
- Single addition of a probiotic preparation of 10⁷ cfu, added at day 9 of the trial. Addition was made in 0.10 ml of MRD (Maximum Recovery Diluent) by cloacal gavage. This method of addition was solely for the purpose to ensure proof of concept. It is not envisaged that this method of addition would be used in a production environment.

Table 1 provides a list of the ingredients in a commercially available poultry feed mixture, with which the composition of the invention may be combined for administration to the poultry.

**Table 1**

| **RM Name** | CONTROL: | CONTROL: | CONTROL: |
|---|---|---|---|
| | ROSS 308 BROILER 2015 - STARTER | ROSS 308 BROILER 2015 - GROWER | ROSS 308 BROILER 2015-FINISHER |
| WHEAT | 59.999 | 60.716 | 66.319 |
| EXT. HIPRO SOYA MEAL | 32.5 | 30.8 | 25.3 |
| LIMESTONE GRANULES | 0.60 | 0.40 | 0.40 |
| SOYABEAN OIL | 3.65 | 5.52 | 5.60 |
| LYSINE HCL | 0.296 | 0.119 | 0.123 |
| METHIONINE DL | 0.362 | 0.263 | 0.231 |
| DICALCIUM PHOSPHATE | 1.59 | 1.28 | 1.12 |
| SODIUM BICARBONATE | 0.269 | 0.188 | 0.193 |
| SALT | 0.150 | 0.210 | 0.210 |
| THREONINE | 0.134 | 0.054 | 0.054 |
| TM - Blank Premix for Broiler | | | |
| Formulation | 0.400 | 0.400 | 0.400 |
| RONOZYME® P5000 (CT) | 0.030 | 0.030 | 0.030 |
| Ronozyme® WX (Xyl) | 0.020 | 0.020 | 0.020 |

| | | | |
|---|---|---|---|
| Key | | | |

- RM: - Raw material
- Ext. Hipro Soya Meal: - Extruded Hipro soya meal (and extruded high protein soybean meal)
- Lysine HCI: - (lysine hydrochloride) and Methionine DL (a racemic mixture of the methionine D and L isomers) amino acids
- Threonine: - an amino acid

### Dicalcium phosphate, sodium bicarbonate, and salt (sodium chloride) are commonly used nutrients

TM - Blank Premix for Broiler Formulation is the premix of vitamins and trace elements listed in Table 2. Ronozyme® P5000 (CT) and Ronozyme® WX (Xyl) are commercial names for enzymes that are commonly used in wheat-based feeds, specifically:
∘ Ronozyme® P5000 (CT) is a coated phytase enzyme
∘ Ronozyme® WX is a xylanase

Reference is also made to *Aviagen Ross 308 Broiler Nutrition Specifications 2014* documentation as examples of broiler diets

Table 2 provides the details of the TM Blank Premix for Broiler Formation listed in the ingredients in Table 1.

**Table 2**

| **Nutrient** | **Analysis** |
|---|---|
| | |
| USAGE | 4.0000 |
| VIT A | 13.5000 |
| VIT D3 | 5.0000 |
| VIT E | 100.0000 |
| VIT B1 | 3.0000 |
| VIT B2 | 10.0000 |
| VIT B6 | 3.0000 |
| VIT B12 | 30.0000 |
| HETRA | 5.0000 |
| NICO | 60.0000 |
| PANTO | 15.0000 |
| FOLIC | 1.5000 |
| BIOTIN | 251.0000 |
| CHOLCHL | 250.0000 |
| FE | 20.0000 |
| MN | 100.0000 |
| CU | 10.0000 |
| ZN | 80.0000 |
| I | 1.0000 |
| SE | 0.2500 |
| MO | 0.5000 |
| *CA/USA | 24.9103 |
| *ASH/USA | 74.3901 |

Tables 3 - 8 provide information regarding a trial experiment (Trial 1) carried out by the Applicant. Trial 1 concerned the performance and the up-regulation of certain commensal bacteria in GOS test treatments.

### Trial 1

### Trial design, measures and analysis

- **Objective(s):** Indicate optimum %(w/w) inclusion rate of galacto-oligosaccharides, reduce and vary the galacto-oligosaccharides %(w/w) inclusion rate in the different feed periods over the lifetime of the bird. The initial objective of the trial was to investigate the effect of Nutrabiotic™ GOS L on the broiler microbiota by NGS and metagenomic analysis (along with analyses of gut morphology and changes in immune function response).
   ∘ Samples for the analysis of gut morphology are stored in formaldehyde awaiting analysis.
   ∘ Results from NGS and metagenomic analysis of the caecal microbiota, along with changes in immune function response (as determined through the up/down regulation of cytokines and chemokines) will be available in the coming weeks.
- **Product:** Nutrabiotic® GOS L - a GOS 50% syrup containing approximately 72 %(w/w) dry solids
- **Base diet:** wheat-based (xylanase and phytase included), no coccidiostat
- **Type of bird:** male Ross 308 (good chicks from strong 35 week old breeders)
- **Number of treatments:** 1 x control + 5 x GOS tests

### Quality Control

- Feed screened for *Salmonella* prior to arrival of birds to ensure no contamination at feed mill.
- Birds screened on arrival for *Salmonella* and during the trial for both *Campylobacter* and *Salmonella.*

### Relevant facts/observations

### General

• Bird health was good with one bird suffering from hip dislocation and another suffering from sudden death. No comments were received concerning gut lesions.

### Performance

- Nutrabiotic™ GOS L improved performance in terms of rate of weight gain with overall the best performance appearing to be for the higher GOS inclusion rate being fed throughout the growth period (P < 0.05). These improvements are maintained in the Test treatments.
   ∘ The confidence intervals of the weight data are quite wide, especially when sample numbers are decreased.
- It appears Nutrabiotic™ GOS L improves FCR for all treatments.

### Standard microbiological analyses

- Standard microbiological methods were used to analyse on caecal samples, by standard microbiological methods:
   *∘ Campylobacter* counts (CCDA plates, micro-aerobic incubation at 42 °C for 48 h, using the Miles and Misra method);
   ∘ lactic bacteria counts (MRS plates, anaerobic incubation at 30 °C for 48 h);
   ∘ coliform counts (MacConkey no.3 plates, incubation at 37 °C for 24 h).

### Microbiota analyses

- DNA was extracted from the caecal microbiota, targeted amplicon sequencing was employed using 16S RDNA (the gene for bacterial 16S rRNA) as a marker and molecular phylogenetic methods (amplification, sequencing, grouping sequences into OTUs, and the identification of OTUs) are used to infer the composition of the microbial community.
- Alpha-diversity (number or richness) of taxa were quantified by the Simpson Index for each treatment with good precision (as shown by asymptotic rarefaction curves) and showed no difference between each treatment, as was expected.
- Beta-diversity, which describes how many taxa are shared between treatments (a similarity score and represented by the Yue and Clayton theta similarity coefficient), gave different results:
   ∘there was a significant difference (P < 0.0010) was found between the GOS[+] and GOS[-] groups (taken as a whole); ;
   ∘ other measures, including AMOVA (analysis of molecular variance) confirmed these significant differences with the magnitude of the diversity being: GOS 3.37% > GOS 1.685% > control with corresponding significance: (GOS 3.37% - GOS 1.685%) > (GOS 1.685% - control);
   ∘ graphical representation of dissimilarities were shown as non-metric multidimensional scaling plots based on dissimilarity matrices built from the Yue and Clayton theta coefficients.
- Metastats (White *et al.,* 2009) was used to determine whether there are any OTUs that are differentially represented between the different treatments:
   ∘ between the GOS[+] and GOS[-] groups (taken as a whole) 42 OTUs were identified as significant.

### Subsequent bioinformatics analyses

The major different OTUs in GOS[+] and GOS[-] groups have been identified, with the following candidate organisms identified as being GOS responsive. Identification was based on OTUs identified from 16S rDNA sequences from the V4 region of the microbiome. It is not possible to obtain more information of exact bacterial subspecies, and in some cases species, without a more complete analysis of the specific bacterial genome. The identification provided represents the nearest match from the SILVA rRNA database (16S rRNA gene sequences were aligned against a reference alignment based on the SILVA rRNA database and clustered into operational taxonomic units (OTUs) with an average neighbor clustering algorithm. The nearest 16S rRNA gene sequence identities to the OTUs are reported on the basis of BLASTn searches if data matches are from type cultures with a BLAST identity ≥99%.):
- *Bifidobacterium animalis* subsp. *lactis* str. V9
- *Collinsella tanakaei* str. YIT 12064
- *Ruminococcus torques* str. ATCC 27756
- *Lactobacillus reuteri* str. BCS136
- *Anaerostipes* sp. str. 35-7
- *Lactobacillus crispatus* str. ST1
- *Pediococcus acidilactici*
- *Faecalibacterium prausnitzii*

### Conclusions

In conclusion it was shown that:
- there was an improvement in performance data, against the control, was seen in test treatments containing Nutrabiotic® GOS Syrup, particularly at the higher dose rate of 3.37 %(w/w);
- there was no significant difference between the "richness" of taxa (alpha-diversity) for each treatment, which is to be expected;
- there was a significant different between the number of taxa shared between between groups (beta-diversity) based on the inclusion of GOS in the diet. this allowed identification of bacteria that were "responsive to Nutrabiotic® GOS.

Table 3 provides a list of ingredients used in a poultry feed as part of Trial 1

**Table 3**

| | | CONTROL: ROSS 308 BROILER 2015 STARTER | CONTROL: ROSS 308 BROILER 2015 GROWER | CONTROL: ROSS 308 BROILER 2015 FINISHER | GOS 3.370%: ROSS 308 BROILER 2015 STARTER | GOS 3.370%: ROSS 308 BROILER 2015 GROWER | GOS 3.370%: ROSS 308 BROILER 2015 FINISHER | GOS 1.685%: ROSS 308 BROILER 2015 STARTER | GOS 1.685%: ROSS 308 BROILER 2015 GROWER | GOS 1.685%: ROSS 308 BROILER 2015 FINISHER |
|---|---|---|---|---|---|---|---|---|---|---|
| 2 | WHEAT | 59.999 | 60.716 | 66.319 | 54.016 | 54.723 | 60.337 | 57.003 | 57.719 | 63.324 |
| 54 | EXT. HIPRO SOYA MEAL | 32.5 | 30.8 | 25.3 | 33.9 | 32.2 | 26.7 | 33.2 | 31.5 | 26.0 |
| 60 | LIMESTONE GRANULES | 0.60 | 0.40 | 0.40 | 0.60 | 0.40 | 0.40 | 0.60 | 0.40 | 0.40 |
| 69 | SOYABEAN OIL | 3.65 | 5.52 | 5.60 | 4.88 | 6.76 | 6.84 | 4.27 | 6.14 | 6.22 |
| 106 | LYSINE HCL | 0.296 | 0.119 | 0.123 | 0.264 | 0.087 | 0.092 | 0.280 | 0.103 | 0.107 |
| 107 | METHIONINE DL | 0.362 | 0.263 | 0.231 | 0.366 | 0.267 | 0.234 | 0.364 | 0.265 | 0.232 |
| 110 | DICALCIUM PHOSPHATE | 1.59 | 1.28 | 1.12 | 1.61 | 1.30 | 1.14 | 1.60 | 1.29 | 1.13 |
| 126 | SODIUM BICARBONATE | 0.269 | 0.188 | 0.193 | 0.249 | 0.169 | 0.173 | 0.259 | 0.179 | 0.183 |
| 273 | SALT | 0.150 | 0.210 | 0.210 | 0.170 | 0.230 | 0.220 | 0.160 | 0.220 | 0.220 |
| 275 | THREONINE | 0.134 | 0.054 | 0.054 | 0.125 | 0.044 | 0.044 | 0.129 | 0.049 | 0.049 |
| TMB LANK | TM - Blank Premix for Broiler Formulation | 0.400 | 0.400 | 0.400 | 0.400 | 0.400 | 0.400 | 0.400 | 0.400 | 0.400 |
| **TM_ PROM OV** | **NUTRABIOTIC GOS SYRUP** | **0.000** | **0.000** | **0.000** | **3.370** | **3.370** | **3.370** | **1.685** | **1.685** | **1.685** |
| TM_RONO_P5 | RONOZYME P5000 (CT) | 0.030 | 0.030 | 0.030 | 0.030 | 0.030 | 0.030 | 0.030 | 0.030 | 0.030 |
| TM_RONO_WX | Ronozyme WX (Xyl) | 0.020 | 0.020 | 0.020 | 0.020 | 0.020 | 0.020 | 0.020 | 0.020 | 0.020 |
| | **Specification** | | | | | | | | | |
| | [VOLUME] | 100 | 100 | 100 | 100 | 100 | 100 | 100 | 100 | 100 |
| | Dry matter | 88.105 | 88.189 | 88.066 | 87.705 | 87.790 | 87.667 | 87.905 | 87.990 | 87.867 |
| | Oil 'B' | 5.707 | 7.526 | 7.566 | 6.844 | 8.672 | 8.713 | 6.280 | 8.099 | 8.139 |
| | Crude Protein (CP) (%) | 22.002 | 21.004 | 19.019 | 21.991 | 20.990 | 19.008 | 21.995 | 20.997 | 19.012 |
| | Fibre (%) | 2.775 | 2.745 | 2.749 | 2.637 | 2.608 | 2.611 | 2.706 | 2.677 | 2.680 |
| | Ash (%) | 5.808 | 5.236 | 4.858 | 5.810 | 5.239 | 4.850 | 5.809 | 5.238 | 4.859 |
| | Lysine (%) | 1.430 | 1.240 | 1.091 | 1.429 | 1.239 | 1.091 | 1.429 | 1.239 | 1.091 |
| | Methionine (%) | 0.691 | 0.582 | 0.521 | 0.695 | 0.586 | 0.524 | 0.693 | 0.584 | 0.522 |
| | Methionine + Cystine (M+C) (%) | 1.070 | 0.950 | 0.861 | 1.070 | 0.950 | 0.860 | 1.070 | 0.950 | 0.860 |
| | Tryptophan (%) | 0.270 | 0.261 | 0.235 | 0.271 | 0.262 | 0.236 | 0.270 | 0.261 | 0.235 |
| | Theonine (%) | 0.940 | 0.830 | 0.741 | 0.940 | 0.829 | 0.740 | 0.939 | 0.830 | 0.740 |
| | Calcium (%) | 1.047 | 0.886 | 0.834 | 1.052 | 0.892 | 0.839 | 1.050 | 0.889 | 0.836 |
| | Total Phosphorus (T:PHOS) (%) | 0.677 | 0.613 | 0.565 | 0.672 | 0.607 | 0.560 | 0.675 | 0.610 | 0.562 |
| | Available Phosphorus (A:PHOS) (%) | 0.500 | 0.450 | 0.420 | 0.500 | 0.450 | 0.420 | 0.500 | 0.450 | 0.420 |
| | Salt (%) | 0.319 | 0.322 | 0.326 | 0.323 | 0.326 | 0.321 | 0.321 | 0.324 | 0.328 |
| | Sodium (%) | 0.158 | 0.160 | 0.161 | 0.160 | 0.162 | 0.159 | 0.159 | 0.161 | 0.162 |
| | Linoleic acid (%) | 2.318 | 3.251 | 3.302 | 2.903 | 3.840 | 3.891 | 2.613 | 3.545 | 3.597 |
| | Potassium (%) | 0.955 | 0.920 | 0.822 | 0.962 | 0.927 | 0.829 | 0.958 | 0.924 | 0.825 |
| | Chloride (%) | 0.198 | 0.200 | 0.201 | 0.201 | 0.202 | 0.198 | 0.199 | 0.201 | 0.203 |
| | Broiler ME inc. enzyme contribution (MJ) | 12.652 | 13.204 | 13.403 | 12.649 | 13.203 | 13.404 | 12.652 | 13.204 | 13.403 |

| | **Degussa poultry digestible amino acid values** | | | | | | | | | |
|---|---|---|---|---|---|---|---|---|---|---|
| | Lysine (%) | 1.306 | 1.122 | 0.984 | 1.305 | 1.121 | 0.984 | 1.306 | 1.122 | 0.984 |
| | Methionine (%) | 0.635 | 0.528 | 0.473 | 0.638 | 0.531 | 0.475 | 0.636 | 0.529 | 0.474 |
| | Methionine + Cystine (M+C) (%) | 0.949 | 0.834 | 0.761 | 0.947 | 0.832 | 0.757 | 0.948 | 0.833 | 0.759 |
| | Theonine (%) | 0.790 | 0.686 | 0.614 | 0.788 | 0.683 | 0.611 | 0.788 | 0.684 | 0.613 |
| | Tryptophan (%) | 0.239 | 0.230 | 0.205 | 0.240 | 0.232 | 0.207 | 0.240 | 0.231 | 0.206 |
| | Isoleucine (%) | 0.814 | 0.785 | 0.703 | 0.820 | 0.790 | 0.708 | 0.817 | 0.788 | 0.705 |
| | Valine (%) | 0.874 | 0.843 | 0.759 | 0.877 | 0.847 | 0.762 | 0.875 | 0.845 | 0.760 |
| | Histidine (%) | 0.496 | 0.479 | 0.429 | 0.499 | 0.481 | 0.432 | 0.497 | 0.480 | 0.431 |
| | Arginine (%) | 1.275 | 1.225 | 1.077 | 1.293 | 1.243 | 1.095 | 1.284 | 1.234 | 1.086 |

Table 4 provides a comparison of the difference in speciation and Degussa poultry digestible amino acid values from Table 3

**Table 4**

| | **Differences** | | | | | |
|---|---|---|---|---|---|---|
| **Specification** | Control - GOS_3.370% diets | | | Control - GOS_1.685% diets | | |
| [VOLUME] | Starter | Grower | Finisher | Starter | Grower | Finisher |
| Dry matter | 0.39934 | 0.39814 | 0.39946 | 0.19913 | 0.19901 | 0.19925 |
| Oil 'B' | 1.13654 | 1.14624 | 1.14646 | 0.57313 | 0.57311 | 0.57315 |
| Crude Protein (CP) (%) | 0.011505 | 0.013285 | 0.01178 | 0.00659 | 0.006695 | 0.007075 |
| Fibre (%) | 0.13749 | 0.13779 | 0.13746 | 0.06888 | 0.06891 | 0.06885 |
| Ash (%) | 0.002628 | 0.003062 | 0.007266 | -0.00123 | 0.001832 | -0.00124 |
| Lysine (%) | 0.001166 | 0.001197 | 0.000373 | 0.000597 | 0.0006 | 0.000594 |
| Methionine (%) | 0.003729 | 0.003712 | 0.002741 | 0.001857 | 0.001855 | 0.000869 |
| Methionine + Cystine (M+C) (%) | 0.000328 | 0.000288 | 0.000658 | 0.000146 | 0.000142 | 0.00084 |
| Tryptophan (%) | 0.000921 | 0.000909 | 0.000922 | 0.000455 | 0.000454 | 0.000456 |
| Theonine (%) | 0.000161 | 0.000859 | 0.000826 | 0.000428 | 0.000431 | 0.000425 |
| Calcium (%) | 0.005708 | 0.005704 | 0.005709 | 0.002852 | 0.002852 | 0.002852 |
| Total Phosphorus (T:PHOS) (%) | 0.005249 | 0.005279 | 0.005246 | 0.002638 | 0.002641 | 0.002635 |
| Available Phosphorus (A:PHOS) (%) | 0.000123 | 0.000133 | 0.000122 | 6.6E-05 | 6.7E-05 | 6.5E-05 |
| Salt (%) | 0.004309 | 0.004299 | 0.005271 | -0.00215 | 0.002149 | 0.002151 |
| Sodium (%) | 0.001888 | 0.002156 | 0.001982 | 0.000943 | 0.001213 | 0.000943 |
| Linoleic acid (%) | 0.584842 | 0.589782 | 0.589848 | 0.294894 | 0.294888 | -0.2949 |
| Potassium (%) | 0.006868 | 0.006828 | 0.006872 | 0.003416 | 0.003412 | -0.00342 |
| Chloride (%) | 0.002923 | 0.002916 | 0.002963 | 0.001459 | 0.001457 | 0.001459 |
| Broiler ME inc. enzyme contribution (MJ) | 0.002985 | 0.000741 | 0.000661 | 0.000305 | 0.000436 | 0.000379 |

| **Degussa poultry digestible amino acid values** | | | | | | |
|---|---|---|---|---|---|---|
| Lysine (%) | 0.000829 | 0.000855 | 4.8E-05 | 0.000426 | 0.000429 | 0.000423 |
| Methionine (%) | -0.00298 | 0.002964 | 0.001988 | 0.001484 | 0.001481 | 0.000491 |
| Methionine + Cystine (M+C) (%) | 0.002343 | 0.00238 | 0.003333 | 0.001188 | 0.001192 | 0.002178 |
| Theonine (%) | 0.002053 | 0.00307 | 0.003043 | 0.001534 | 0.001536 | 0.001532 |
| Tryptophan (%) | 0.001717 | 0.001707 | 0.001718 | 0.000854 | 0.000853 | 0.000855 |
| Isoleucine (%) | -0.0051 | 0.005064 | 0.005104 | 0.002535 | - 0.00253 | 0.002538 |
| Valine (%) | 0.003368 | 0.003328 | 0.003372 | 0.001666 | 0.001662 | - 0.00167 |
| Histidine (%) | - | - | - | - | - | - |
| | 0.002517 | 0.002496 | 0.002519 | 0.001249 | 0.001247 | 0.001251 |
| Arginine (%) | 0.018093 | - 0.01805 | 0.018097 | 0.009027 | 0.009023 | 0.009031 |

Table 5 provides a summary of the treatments used in Trial 1

**Table 5**

| | | | | | | | | |
|---|---|---|---|---|---|---|---|---|
| **Group** | **1** | Control | | starter | 1 | to | 10 | days |
| | | Control | | grower | 11 | to | 24 | days |
| | | Control | | finisher | 25 | to | 35 | days |
| **Group** | **2** | 3.37 %(w/w) GOS | | starter | 1 | to | 10 | days |
| | | Control feed | | grower | 11 | to | 24 | days |
| | | Control feed | | finisher | 25 | to | 35 | days |
| **Group** | **3** | 3.37 %(w/w) GOS | | starter | 1 | to | 10 | days |
| | | 3.37 %(w/w) GOS | | grower | 11 | to | 24 | days |
| | | Control feed | | finisher | 25 | to | 35 | days |
| **Group** | **4** | 3.37 %(w/w) GOS | | starter | 1 | to | 10 | days |
| | | 3.37 %(w/w) GOS | | grower | 11 | to | 24 | days |
| | | 3.37 %(w/w) GOS | | finisher | 25 | to | 35 | days |
| **Group** | **5** | Control feed | | starter | 1 | to | 10 | days |
| | | Control feed | | grower | 11 | to | 24 | days |
| | | 3.37 %(w/w) GOS | | finisher | 25 | to | 35 | days |
| **Group** | **6** | 1.685 %(w/w) GOS | | starter | 1 | to | 10 | days |
| | | 1.685 %(w/w) GOS | | grower | 11 | to | 24 | days |
| | | 1.685 %(w/w) GOS | | finisher | 25 | to | 35 | days |

Table 6 provides the weight (g) of the broilers used in Trial 1

**Table 6**

| **Group** | **Weight (g)** | | | | | | | | |
|---|---|---|---|---|---|---|---|---|---|
| | **0** | **8** | **15** | **22** | **28** | **35** | **Days** | | |
| G1 | **40.8** | **180.0** | **498.7** | **934.5** | **1411.5** | **2012.0** | **Total** | (g) | **mean** |
| | 2.87 | 13.61 | 56.25 | 105.33 | 176.08 | 213.64 | | | stdev |
| G2 | **40.9** | **188.0** | **556.4** | **1032.9** | **1623.7** | **2270.7** | **Total** | (g) | **mean** |
| | 2.85 | 13.76 | 61.16 | 138.65 | 168.04 | 253.01 | | | stdev |
| G3 | **40.9** | **190.1** | **531.6** | **1009.1** | **1554.1** | **2126.6** | **Total** | (g) | **mean** |
| | 2.96 | 15.80 | 54.51 | 119.53 | 190.86 | 210.33 | | | stdev |
| G4 | **41.3** | **183.5** | **562.5** | **1030.5** | **1608.8** | **2360.8** | **Total** | (g) | mean |
| | 2.88 | 14.97 | 64.93 | 120.45 | 155.29 | 144.36 | | | stdev |
| G5 | **40.2** | **185.2** | **517.0** | **923.1** | **1491.1** | **2197.5** | **Total** | (g) | mean |
| | 2.71 | 20.25 | 58.53 | 127.35 | 165.93 | 344.96 | | | stdev |
| G6 | **40.5** | **187.9** | **526.2** | **974.8** | **1540.0** | **2173.5** | **Total** | (g) | mean |
| | 3.14 | 22.36 | 57.52 | 119.99 | 170.21 | 216.79 | | | stdev |

Table 7 provides the feed consumption of the broilers used in Trial 1

**Table 7**

| **Group** | **Feed consumption (g)** | | | | | | | | | | |
|---|---|---|---|---|---|---|---|---|---|---|---|
| | **0** | **10** | **15** | **22** | **25** | **28** | **35** | **Days** | | | |
| G1 | | **230** | **273** | **657** | **411** | **453** | **1093** | **Total** | **Interval** | (g) | **mean** |
| | | 12 | 39 | 86 | 70 | 109 | 117 | | | | stdev |
| | | **230** | **503** | **1160** | **1571** | **2024** | **3117** | | **Cumm.** | (g) | |
| G2 | | **230** | **305** | **725** | **452** | **500** | **1209** | **Total** | **Interval** | (g) | mean |
| | | 10 | 26 | 90 | 35 | 85 | 102 | | | | stdev |
| | | **230** | **535** | **1260** | **1712** | **2212** | **3421** | | **Cumm.** | (g) | |
| G3 | | **236** | **270** | **713** | **415** | **468** | **1292** | **Total** | **Interval** | (g) | **mean** |
| | | 13 | 33 | 82 | 62 | 92 | 71 | | | | stdev |
| | | **236** | **506** | **1219** | **1634** | **2102** | **3394** | | **Cumm.** | (g) | |
| G4 | | **224** | **300** | **740** | **461** | **515** | **1356** | **Total** | **Interval** | (g) | **mean** |
| | | 8 | 22 | 82 | 59 | 69 | 55 | | | | stdev |
| | | **224** | **524** | **1264** | **1725** | **2240** | **3596** | | **Cumm.** | (g) | |
| G5 | | **222** | **289** | **717** | **428** | **495** | **1225** | **Total** | **Interval** | (g) | **mean** |
| | | 12 | 32 | 84 | 58 | 93 | 105 | | | | stdev |
| | | **222** | **511** | **1228** | **1656** | **2151** | **3376** | | **Cumm.** | (g) | |
| G6 | | **238** | **241** | **719** | **450** | **502** | **1188** | **Total** | **Interval** | (g) | **mean** |
| | | 18 | 42 | 76 | 56 | 91 | 54 | | | | stdev |
| | | **238** | **479** | **1198** | **1648** | **2150** | **3338** | | **Cumm.** | (g) | |

Table 8 provides the cumulative feed consumption ratio of the broilers used in Trial 1

**Table 8**

| **Group** | **Weight (g)** | | | | | | |
|---|---|---|---|---|---|---|---|
| | **0** | **8** | **15** | **22** | **28** | **35** | **Days** |
| G1 | | 0.890 | 1.000 | 1.240 | 1.434 | 1.549 | **Total** |
| G2 | | 0.850 | 0.962 | 1.220 | 1.363 | 1.507 | **Total** |
| G3 | | 0.870 | 0.953 | 1.210 | 1.353 | 1.596 | **Total** |
| G4 | | 0.860 | 0.948 | 1.220 | 1.393 | 1.524 | **Total** |
| G5 | | 0.850 | 0.990 | 1.330 | 1.442 | 1.537 | **Total** |
| G6 | | 0.880 | 0.897 | 1.230 | 1.396 | 1.536 | **Total** |

Tables 9 - 20 provide information regarding a trial experiment (Trial 2) carried out by the Applicant. Trial 2 concerned the use of *Lactobacillus crispatus* DC21.1 (NCIMB 42771) as a probiotic

### Trial 2

### Objectives

To test the persistence and efficacy of *Lactobacillus crispatus* was provided as a probiotic to male Ross 308 broilers fed a standard wheat-based feed in the presence and absence of the galacto-oligosaccharide contain product - Nutrabiotic® GOS.

### Design

4 treatments each containing 20 -24 male Ross 308 broiler that were fed a standard wheat-based starter, grower and finisher feed. The feeds contained no antibiotic or coccidiostat products, but Nutrabiotic® GOS and *Lactobacillus crispatus* DC21.1 (NCIMB 42771). Details of the feed are given below and in the associated files. The trial was carried out for 35 days, and the *Lactobacillus crispatus* was added on day 9 by cloacal gavage with 10⁷ cfu (viable cells) being administered in 0.10 ml MRD (maximum recovery diluent) from a syringe that had been preloaded in an anerobic cabinet.

| | | | |
|---|---|---|---|
| • Group 1: | Pen 6 | Nutrabiotic® GOS | *Lactobacillus crispatus* |
| • Group 2: | Pen 7 | Nutrabiotic® GOS | not added |
| • Group 3: | Pen 8 | not added | not added |
| • Group 4: | Pen 9 | not added | *Lactobacillus crispatus* |

### Results

There was only a single addition of the *Lactobacillus crispatus* was added on day 9 after bird placement. Persistence of the *Lactobacillus crispatus* was determined as follows:
- DNA extractions were made from from caeca contents (MPBio kit) with concentration ranges of 80-250 ng/µl;
- DNA concentrations were normalised;
- qPCR was used, with absolute quantification using a standard curve based on extracted *Lactobacillus crispatus* DNA at different dilutions.

The concentration of the *Lactobacillus crispatus,* which is a commensal strain, when administered on day 9 after bird placement was present at the end of the trial at 1.9 - 2.9 x the concentration in treatments where it had not been added by oral gavage.

Whilst this was not a large trial, lacking statistical power, and the results were not significant in that P > 0.05, the increase in bird weight at 35 days was greatest for group 1 (Nutrabiotic® GOS + *Lactobacillus crispatus*) with, in some comparisons P < 0.10.

### Conclusions

*Lactobacillus crispatus* DC21.1 (NCIMB 42771) persists in the broiler caecum at the end of the experiment period, at day 35, when administered at day 9. The probiotic was present a concentrations of 1.9 - 2.9 x the concentration in control treatments. Whilst the trial lacked statistical power, and the results were not significant in that P > 0.05, the increase in bird weight at 35 days was greatest the test group (Nutrabiotic® GOS + *Lactobacillus crispatus*) with, in some comparisons P < 0.10.

**Table 9**

| Table 9 provides the performance data of Trial 2 - Group 1, Pen 6 | | | | | | | |
|---|---|---|---|---|---|---|---|
| **Group 1** | **Pen 6** | **GOS2** | ***Lactobacillus crispatus*** | | | | |
| Date | | 08/11/16 | 15/11/16 | 18/11/16 | 28/11/16 | 05/12/16 | 13/12/16 |
| Time | (days) | 0 | 7 | 10 | 20 | 27 | 35 |

| Bird | | **Weight** | **Weight** | **Weight** | **Weight** | **Weight** | **Weight** |
|---|---|---|---|---|---|---|---|
| | | (g) | (g) | (g) | (g) | (g) | (g) |
| 1 | | 41 | 214 | 262 | 740 | | |
| 2 | | 40 | 198 | 345 | 985 | 1321 | 1931 |
| 3 | | 35 | 189 | 323 | 803 | | |
| 4 | | 39 | 198 | 313 | 876 | 1159 | 1745 |
| 5 | | 38 | 219 | 332 | 937 | 1432 | 2270 |
| 6 | | 37 | 181 | 297 | 715 | | |
| 7 | | 40 | 183 | 282 | 758 | 1298 | 1803 |
| 8 | | 41 | 185 | 302 | 792 | 1379 | 2240 |
| 9 | | 34 | 218 | 299 | 782 | | |
| 10 | | 41 | 171 | 292 | 742 | 1240 | 1958 |
| 11 | | 43 | 173 | 313 | 925 | 1202 | 1998 |
| 12 | | 38 | 191 | 272 | 746 | | |
| 13 | | 35 | 186 | 276 | 717 | | |
| 14 | | 39 | 203 | 230 | 862 | | |
| 15 | | 39 | 196 | 298 | 867 | | |
| 16 | | 37 | 147 | 204 | 617 | | |
| 17 | | 39 | 177 | 263 | 862 | 1372 | 2260 |
| 18 | | 41 | 171 | 319 | 986 | 1361 | 2080 |
| 19 | | 40 | 196 | 330 | 828 | 1294 | 2040 |
| 20 | | 41 | 149 | 251 | 779 | | |
| | **average weight** | **38.9** | **187.3** | **290.2** | **816.0** | **1305.8** | **2032.5** |
| | st. dev. | 2.4 | 19.6 | 35.8 | 96.5 | 85.5 | 184.4 |
| | RSD (%) | 6.1% | 10.5% | 12.3% | 11.8% | 6.5% | 9.1% |
| | cum. feed per bird (g) | | 163 | 339 | 1023 | 1896 | 3196 |
| | **FCR** | | **0.870** | **1.168** | **1.254** | **1.452** | **1.572** |

Table 10 provides the performance data of Trial 2 - Group 2, Pen 7

**Table 10**

| **Group 2** | **Pen 7** | **GOS2** | | | | | |
|---|---|---|---|---|---|---|---|
| Date | | 08/11/16 | 15/11/2016 | 18/11/2016 | 28/11/2016 | 05/12/2016 | 13/12/2016 |
| Time | (days) | 0 | 7 | 10 | 20 | 27 | 35 |

| Bird | | **Weight** | **Weight** | **Weight** | **Weight** | **Weight** | **Weight** |
|---|---|---|---|---|---|---|---|
| | | (g) | (g) | (g) | (g) | (g) | (g) |
| 1 | | 36 | 178 | 295 | 782 | | |
| 2 | | 33 | 148 | 248 | 621 | | |
| 3 | | 35 | 179 | 262 | 757 | 1042 | 1567 |
| 4 | | 35 | 148 | 234 | 628 | | |
| 5 | | 38 | 179 | 256 | 799 | 1317 | 2018 |
| 6 | | 40 | 189 | 254 | 892 | | |
| 7 | | 41 | 168 | 256 | 766 | 1112 | 1787 |
| 8 | | 41 | 175 | 263 | 792 | 1282 | 1946 |
| 9 | | 38 | 168 | 299 | 717 | | |
| 10 | | 38 | 172 | 275 | 778 | | |
| 11 | | 41 | 164 | 329 | 788 | | |
| 12 | | 37 | 161 | 308 | 746 | 1082 | 1632 |
| 13 | | 38 | 166 | 282 | 781 | | |
| 14 | | 39 | 201 | 320 | 862 | 1192 | 1769 |
| 15 | | 40 | 197 | 324 | 898 | 1186 | 1738 |
| 16 | | 39 | 154 | 294 | 728 | 1132 | 1670 |
| 17 | | 38 | 166 | 242 | 719 | | |
| 18 | | 43 | 207 | 276 | 986 | 1372 | 2149 |
| 19 | | 42 | 204 | 355 | 978 | 1524 | 2289 |
| 20 | | 40 | 176 | 251 | 779 | | |
| | **average weight** | **38.6** | **175.0** | **281.2** | **789.9** | **1224.1** | **1856.5** |
| | st. dev. | 2.5 | 17.4 | 33.3 | 95.7 | 149.3 | 236.2 |
| | RSD (%) | 6.6% | 9.9% | 11.8% | 12.1% | 12.2% | 12.7% |
| | cum. feed per bird (g) | | 158 | 336 | 939 | 1699 | 2945 |
| | **FCR** | | **0.900** | **1.193** | **1.189** | **1.388** | **1.586** |

Table 11 provides the performance data of Trial 2 - Group 3, Pen 8

**Table 11**

| **Group 3** | **Pen 8** | | | | | | |
|---|---|---|---|---|---|---|---|
| Date | | 08/11/201 6 | 15/11/201 6 | 18/11/201 6 | 28/11/201 6 | 05/12/201 6 | 13/12/201 6 |
| Time | (days) | 0 | 7 | 10 | 20 | 27 | 35 |
| Bird | | **Weight** | **Weight** | **Weight** | **Weight** | **Weight** | **Weight** |
| | | (g) | (g) | (g) | (g) | (g) | (g) |
| 1 | | 40 | 214 | 319 | 900 | 1492 | 2172 |
| 2 | | 43 | 198 | 364 | 985 | 1424 | 2020 |
| 3 | | 39 | 189 | 294 | 658 | 1156 | 1932 |
| 4 | | 39 | 198 | 249 | 626 | 1154 | 2002 |
| 5 | | 36 | 219 | 264 | 626 | 1176 | 1780 |
| 6 | | 37 | 181 | 290 | 705 | | |
| 7 | | 37 | 183 | 264 | 692 | | |
| 8 | | 39 | 185 | 293 | 772 | 1294 | 1789 |
| 9 | | 39 | 218 | 294 | 737 | 1094 | 1693 |
| 10 | | 43 | 171 | 285 | 870 | 1482 | 2109 |
| 11 | | 44 | 173 | 307 | 930 | 1374 | 2039 |
| 12 | | 38 | 191 | 256 | 705 | | |
| 13 | | 42 | 186 | 283 | 930 | 1336 | 1720 |
| 14 | | 42 | 203 | 314 | 893 | | |
| 15 | | 38 | 196 | 258 | 753 | 1161 | 1803 |
| 16 | | 41 | 147 | 310 | 857 | | |
| 17 | | 42 | 177 | 303 | 870 | 1294 | 1720 |
| 18 | | 41 | 171 | 287 | 802 | | |
| 19 | | 40 | 196 | 297 | 840 | 1424 | 2123 |
| 20 | | 38 | 169 | 283 | 781 | | |
| 21 | | 37 | 149 | 249 | 589 | | |
| 22 | | 41 | 182 | 287 | 802 | | |
| 23 | | 30 | 190 | 282 | 858 | 1482 | 2163 |
| 24 | | 42 | 171 | 267 | 799 | | |
| | **average weight** | **39.5** | **185.7** | **287.5** | **790.8** | **1310.2** | **1933.2** |
| | st. dev. | 3.0 | 18.5 | 25.8 | 107.3 | 141.0 | 177.6 |
| | RSD (%) | 7.6% | 10.0% | 9.0% | 13.6% | 10.8% | 9.2% |
| | cum. feed per bird (g) | | 158 | 328 | 999 | 1760 | 2932 |
| | **FCR** | | **0.848** | **1.142** | **1.263** | **1.344** | **1.517** |

Table 12 provides the performance data of Trial 2 - Group 4, Pen 9

**Table 12**

| **Group 4** | **Pen 9** | | ***Lactobacill us crispatus*** | | | | |
|---|---|---|---|---|---|---|---|
| Date | | 08/11/201 6 | 15/11/2016 | 18/11/201 6 | 28/11/201 6 | 05/12/201 6 | 13/12/201 6 |
| Time | (days) | 0 | 7 | 10 | 20 | 27 | 35 |
| Bird | | **Weight** | **Weight** | **Weight** | **Weight** | **Weight** | **Weight** |
| | | (g) | (g) | (g) | (g) | (g) | (g) |
| 1 | | 41 | 214 | 272 | 720 | | |
| 2 | | 40 | 198 | 230 | 715 | 1084 | 1718 |
| 3 | | 35 | 189 | 244 | 739 | | |
| 4 | | 39 | 198 | 315 | 703 | | |
| 5 | | 38 | 219 | 261 | 658 | 1078 | 1700 |
| 6 | | 37 | 181 | 266 | 679 | 1061 | 1676 |
| 7 | | 40 | 183 | 237 | 691 | | |
| 8 | | 41 | 185 | 274 | 668 | 1180 | 1890 |
| 9 | | 34 | 218 | 306 | 714 | 1089 | 1525 |
| 10 | | 41 | 171 | 315 | 802 | | |
| 11 | | 43 | 173 | 287 | 791 | 1324 | 2015 |
| 12 | | 38 | 191 | 343 | 920 | 1422 | 2080 |
| 13 | | 35 | 186 | 350 | 952 | 1548 | 2300 |
| 14 | | 39 | 203 | 330 | 872 | 1361 | 2052 |
| 15 | | 39 | 196 | 290 | 819 | 1248 | 1825 |
| 16 | | 37 | 147 | 272 | 719 | 1261 | 1932 |
| 17 | | 39 | 177 | 263 | 720 | | |
| 18 | | 41 | 171 | 297 | 752 | | |
| 19 | | 40 | 196 | 280 | 742 | 1214 | 1840 |
| 20 | | 41 | 149 | 281 | 779 | | |
| 21 | | 38 | 185 | 309 | 799 | 1312 | 1970 |
| 22 | | 36 | 152 | 244 | 791 | | |
| 23 | | 40 | 188 | 276 | 801 | 1328 | 1890 |
| 24 | | 41 | 207 | 311 | 895 | | |
| | **average weight** | **38.9** | **186.5** | **285.5** | **768.4** | **1250.7** | **1886.6** |
| | st. dev. | 2.3 | 19.7 | 32.3 | 79.6 | 144.4 | 197.2 |
| | RSD (%) | 5.9% | 10.6% | 11.3% | 10.4% | 11.5% | 10.5% |
| | cum. feed per bird (g) | | 158 | 323 | 938 | 1719 | 2872 |
| | **FCR** | | **0.847** | **1.131** | **1.221** | **1.375** | **1.522** |

Table 13 provides the t-Test data from Trial 2

**Table 13**

| **t-Test** | | | | | | | |
|---|---|---|---|---|---|---|---|
| | **Test** | **Time (days)** | | | | | |
| | | 0 | 7 | 10 | 20 | 27 | 35 |

| | | **P-values** | | | | | |
|---|---|---|---|---|---|---|---|
| | Gp 1 vs 2 | 0.7011 | 0.0433 | 0.4151 | 0.3959 | 0.1506 | 0.0797 |
| | Gp 1 vs 3 | 0.4711 | 0.7901 | 0.7737 | 0.4232 | 0.9308 | 0.1974 |
| | Gp 1 vs 4 | 0.9718 | 0.9058 | 0.6559 | 0.0801 | 0.2940 | 0.0802 |

Table 14 provides the feed consumption data from Trial 2 - Group 1, Pen 6

**Table 14**

| **Group 1** | **Pen 6** | **GOS2** | ***Lactobacillus crispatus*** | | | | | |
|---|---|---|---|---|---|---|---|---|
| **Date** | **Age** | **feed start** | **feed end** | **feed consumed** | **cum. feed consumed** | **no of birds** | **feed per bird** | **cum. feed per bird** |
| | (days) | (g) | (g) | (g) | (g) | (g) | (g) | (g) |
| 08/11/2016 | 0 | 4000 | 740 | | | | | |
| 15/11/2016 | 7 | 5000 | 1480 | 3260 | 3260 | 20 | 163 | 163 |
| 18/11/2016 | 10 | 14600 | 920 | 3520 | 6780 | 20 | 176 | 339 |
| 28/11/2016 | 20 | 12000 | 3274 | 13680 | 20460 | 20 | 684 | 1023 |
| 05/12/2016 | 27 | 16000 | 3000 | 8726 | 29186 | 10 | 873 | 1896 |
| 13/12/2016 | 35 | | | 13000 | 42186 | 10 | 1300 | 3196 |

Table 15 provides the feed consumption data from Trial 2 - Group 2, Pen 7

**Table 15**

| **Group 2** | **Pen 7** | **GOS2** | | | | | | |
|---|---|---|---|---|---|---|---|---|
| **Date** | **Age** | **feed start** | **feed end** | **feed consumed** | **cum. feed consumed** | **no of birds** | **feed per bird** | **cum. feed per bird** |
| | (days) | (g) | (g) | (g) | (g) | (g) | (g) | (g) |
| 08/11/2016 | 0 | 4000 | 850 | | | | | |
| 15/11/2016 | 7 | 5000 | 1440 | 3150 | 3150 | 20 | 158 | 158 |
| 18/11/2016 | 10 | 14600 | 2526 | 3560 | 6710 | 20 | 178 | 336 |
| 28/11/2016 | 20 | 12000 | 4400 | 12074 | 18784 | 20 | 604 | 939 |
| 05/12/2016 | 27 | 16000 | 3540 | 7600 | 26384 | 10 | 760 | 1699 |
| 13/12/2016 | 35 | | | 12460 | 38844 | 10 | 1246 | 2945 |

Table 16 provides the feed consumption data from Trial 2 - Group 3, Pen 8

**Table 16**

| **Group 3** | **Pen 8** | | | | | | | |
|---|---|---|---|---|---|---|---|---|
| **Date** | **Age** | **feed start** | **feed end** | **feed consumed** | **cum. feed consumed** | **no of birds** | **feed per bird** | **cum. feed per bird** |
| | (days) | (g) | (g) | (g) | (g) | (g) | (g) | (g) |
| 08/11/2016 | 0 | 4000 | 220 | | | | | |
| 15/11/2016 | 7 | 5000 | 900 | 3780 | 3780 | 24 | 158 | 158 |
| 18/11/2016 | 10 | 16200 | 109 | 4100 | 7880 | 24 | 171 | 328 |
| 28/11/2016 | 20 | 12000 | 1338 | 16091 | 23971 | 24 | 670 | 999 |
| 05/12/2016 | 27 | 20000 | 3599 | 10662 | 34633 | 14 | 762 | 1760 |
| 13/12/2016 | 35 | | | 16401 | 51034 | 14 | 1172 | 2932 |

Table 17 provides the feed consumption data from Trial 2 - Group 4, Pen 9

**Table 17**

| **Group 4** | **Pen 9** | ***Lactobacillus crispatus*** | | | | | | |
|---|---|---|---|---|---|---|---|---|
| **Date** | **Age** | **feed start** | **feed end** | **feed consumed** | **cum. feed consumed** | **no of birds** | **feed per bird** | **cum. feed per bird** |
| | (days) | (g) | (g) | (g) | (g) | (g) | (g) | (g) |
| 08/11/2016 | 0 | 4000 | 210 | | | | | |
| 15/11/2016 | 7 | 5000 | 1040 | 3790 | 3790 | 24 | 158 | 158 |
| 18/11/2016 | 10 | 16200 | 1433 | 3960 | 7750 | 24 | 165 | 323 |
| 28/11/2016 | 20 | 12000 | 1063 | 14767 | 22517 | 24 | 615 | 938 |
| 05/12/2016 | 27 | 20000 | 3865 | 10937 | 33454 | 14 | 781 | 1719 |
| 13/12/2016 | 35 | | | 16135 | 49589 | 14 | 1153 | 2872 |

Table 18 is the feed formulation used in Trial 2, days 0 - 10

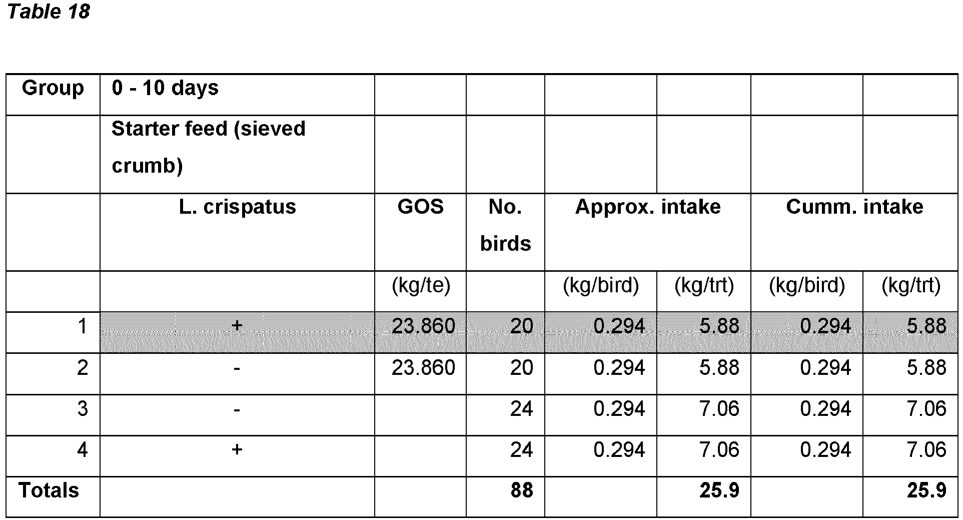

Table 19 provides the feed formulation used in Trial 2, days 11 - 24

**Table 19**

| **Group** | 11-24 days | | | | | | |
|---|---|---|---|---|---|---|---|
| | Grower feed (pellets 3 mm diam.) | | | | | | |
| | L. crispatus | GOS | No. birds | Intake | | Cumm. intake | |
| | | (kg/te) | | (kg/bird) | (kg/trt) | (kg/bird) | (kg/trt) |
| 1 | + | 11.93 | 20 | 1.312 | 26.24 | 1.606 | 32.12 |
| 2 | - | 11.93 | 20 | 1.312 | 26.24 | 1.606 | 32.12 |
| 3 | - | | 24 | 1.312 | 31.488 | 1.606 | 38.544 |
| 4 | + | | 24 | 1.312 | 31.488 | 1.606 | 38.544 |
| **Totals** | | | 88 | | 115.456 | | 141.328 |

Table 20 is the feed formulation used in Trial 2, days 25 - 35

**Table 20**

| **Group** | 25-35 days | | | | | | |
|---|---|---|---|---|---|---|---|
| | Finisher feed (pellets 3 mm diam.) | | | | | | |
| | L. crispatus | GOS | No. birds | Intake | | Cumm. intake | |
| | | (kg/te) | | (kg/bird) | (kg/trt) | (kg/bird) | (kg/trt) |
| 1 | + | 0 | 20 | 1.904 | 38.08 | 3.51 | 70.2 |
| 2 | - | 0 | 20 | 1.904 | 38.08 | 3.51 | 70.2 |
| 3 | - | | 24 | 1.904 | 45.696 | 3.51 | 84.24 |
| 4 | + | | 24 | 1.904 | 45.696 | 3.51 | 84.24 |
| **Totals** | | | 88 | | 167.552 | | 308.88 |

Table 21 provides a description of Nutrabiotic® GOS L with which the composition of the invention may comprise as a prebiotic.

**Table 21**

| Nutrabiotic® GOS L | | |
|---|---|---|
| **Description** : galacto-oligosaccharide syrup. | | |
| **Typical analysis** : dry matter: 75 %(w/w) of which galacto-oligosaccharides: 59 %(w/w DM), lactose: 17%(w/w DM), glucose: 17%(w/w DM), galactose: 7%(w/w DM) | | |
| **Sensorial:** clear yellow to colourless liquid syrup, slightly sweet taste. | | |
| | **Specification** | **Method of analysis** |
| **Chemical and physical:** | | |
| Dry matter | 74 ± 2 %(w/w) | IDF 26A (1993), 2½ h 102±2°C |
| | | |
| Galacto-oligosaccharides | ≥ 57 %(w/w DM) | |
| Lactose | ≤ 23 %(w/w DM) | Dairy Crest methods: |
| Glucose | ≤ 22 %(w/w DM) | C-T.09, issue 01, Aug-2014 |
| Galactose | ≥ 0.8 %(w/w DM) | C-T.10, issue 06, Mar-2016 |
| | | |
| Total Nitrogen | ≤ 0.1 %(w/w DM) | IDF 20B (1993), Kjeldahl |
| Sulphated ash | ≤ 0.3 %(w/w DM) | AOAC 17 ed. (2000) 930.30, sulphated = 550°C to constant weight |
| | | |
| Viscosity | 1000-5000 mPa.s | HAAKE |
| pH | 3.1 - 3.8 | ISO 10523 (1994), potentiometric (10 % w/w) |
| | | |

| *Microbiological:* | | |
|---|---|---|
| Total plate count | ≤ 1000 cfu/g | IDF 100B (1991), PCMA 72h 30°C |
| Yeasts | ≤ 50 cfu/g | IDF 94B (1990), OGYE 5 days 25°C |
| Moulds | ≤ 50 cfu/g | IDF 94B (1990), OGYE 5 days 25°C |
| *Enterobacteriaceae* | absent in 1 g | BDI 23, VRBG 24h 30°C |
| *Escherichia coli* | absent in 5 g | IDF 170A-1 (1999), LSTB 48h 37°C, ECB 48h 44°C |
| *Salmonelleae* | absent in 25 g | IDF 93B (1995) |
| | | |
| ***Packaging:*** | 1200 kg IBC | |
| | | |
| ***Storage:*** | keep in clean, dry and dark conditions, keep away from strongly odorous materials. | |
| ***Shelf life:*** | 18 months after production date. | |

Table 22 provides recommendations based on typical feeding regimes and ones that have been used in both research and commercial trials. They can be modified as required. A comparison between broilers and piglets is also provided.

**Table 22**

| **Batch number** - Nutrabiotic® GOS L batch no. AQ6215 | | |
|---|---|---|
| Dry matter 74.2 %(w/w) | | |
| Water 25.8 %(w/w) | | |
| **Broilers** | | |
| Starter feed | day 0 - 10 | 24.70 kg per metric tonne of complete feed |
| Grower feed | day 11 - 24 | 12.35 kg per metric tonne of complete feed |
| Finisher feed | day 25 - | not generally required |

| **Piglets** | | |
|---|---|---|
| Creep (pre-starter) feed | day 10 - weaning | 15.1 kg per metric tonne of complete feed |
| Weaning | day 28 | |
| Starter feed | day 28 - 35 | 9.1 kg per metric tonne of complete feed |
| Link feed | day 35 - 49 | 9.1 kg per metric tonne of complete feed |
| Grower feed | day 49 - 63 | as required |

### Notes

Dose rates for Nutrabiotic® GOS L are given for the syrup product as is.

Table 23 provides primers sequence 5'-3' for the genes expression determined by qPCR.

**Table 23**

| Target gene | Primer sequence (5'-3') | Product size (bp) | NCBI Accession number | Reference |
|---|---|---|---|---|
| GAPDH | F: GACGTGCAGCAGGAACACTA | 343 | NM 204305.1 | Nang *et al.* (2011) |
| | R: TCTCCATGGTGGTGA AGACA | | | |
| IFN-γ | F: TGAGCCAGATTGTTTCGATG | 152 | NM 205149.1 | Nang *et al.* (2011) |
| | R: CTTGGCCAGGTCCATGATA | | | |
| IL-1β | F: GGATTCTGAGCACACCACAGT | 272 | NM 204524.1 | Nang *et al.* (2011) |
| | R: TCTGGTTGATGTCGAAGATGTC | | | |
| IL-4 | F: GGAGAGCATCCGGATAGTGA | 186 | NM 001007079.1 | Nang *et al.* (2011) |
| | R: TGACGCATGTTGAGGAAGAG | | | |
| IL-10 | F: GCTGCGCTTCTACACAGATG | 203 | NM 001004414.2 | Nang *et al.* (2011) |
| | R: TCCCGTTCTCATCCATCTTC | | | |
| IL-6 | F: GCTCGCCGGCTTCGA | 71 | NM 204628.1 | Kaiser *et al.* (2003) |
| | R: GGTAGGTCTGAAAGGCGAACAG | | | |
| IL17-A | F: CATGGGATTACAGGATCGATGA | 68 | NM 204460.1 | Reid *et al.* (2016) |
| | R: GCGGCACTGGGCATCA | | | |
| IL17-F | F: TGACCCTGCCTCTAGGATGATC | 78 | XM 426223.5 | Reid *et al.* (2016) |
| | R: GGGTCCTCATCGAGCCTGTA | | | |
| ChCXCLi1 | F: CCGATGCCAGTGCATAGAG | 191 | NM 205018.1 | Rasoli *et al.* (2015) |
| | R: CCTTGTCCAGAATTGCCTTG | | | |
| ChCXCLi2 | F: CCTGGTTTCAGCTGCTCTGT | 128 | NM 205498.1 | Rasoli *et al.* (2015) |
| | R: GCGTCAGCTTCACATCTTGA | | | |

Table 24 provides an estimate of the metabolizable energy values of Nutrabiotic® GOS L in broilers and piglets.

**Table 24**

| **Batch number** | |
|---|---|
| Nutrabiotic® GOS L batch no. AQ6215 | |
| Dry matter | 74.2 %(w/w) |
| Water | 25.8 %(w/w) |

**Net Metabolizable Energy (NME): broilers** 6.06 kJ/g Nutrabiotic® GOS L syrup product 1.45 kcal/g Nutrabiotic® GOS L syrup product **Net Metabolizable Energy (NME): piglets** 7.26 kJ/g Nutrabiotic® GOS L syrup product 1.74 kcal/g Nutrabiotic® GOS L syrup product

### Notes

The NME values are expressed per weight of the Nutrabiotic® GOS L product as is, i.e. the syrup product that is added.

It is of course to be understood that the present invention is not intended to be restricted to the foregoing examples which are described by way of example only.

The present invention relates to compositions for use in the treatment and/or nutrition of poultry, such as broiler chickens (*Gallus gallus domesticus*). However it is not beyond the scope of the invention that the present invention may also relate to game birds such as grouse, pheasant or quail, for example.

### ANNEX

1. A composition comprising:
   (iii) a probiotic selected from one or more of the bacteria *Bifidobacterium animalis, Collinsella tanakaei, Lactobacillus reuteri, Anaerostipes, Lactobacillus crispatus, Pediococcus acidilactici, Lactobacillus pontis, Faecalibacterium prausnitzii, Coprococcus catus, Roseburia intestinalis, Anaerostipes butyraticus, Butyricicoccus, Lactobacillus johnsonii, and Ruminococcus* sp.; and
   (iv) a prebiotic material.
2. A composition according to item 1, wherein the one or more bacteria are selected from *Bifidobacterium animalis* subsp. *lactis* str. V9, *Collinsella tanakaei* str. YIT 12064, *Lactobacillus reuteri* str. BCS136, *Anaerostipes* sp. str. 35-7, *Lactobacillus crispatus* str. ST1, *Lactobacillus crispatus* str. DC21, *Lactobacillus crispatus* str. DC21.1 (NCIMB 42771), *Lactobacillus johnsonii* DC22.2 (NCIMB 42772), *Lactobacillus reuteri* DC1B4 (NCIMB 42773), and *Ruminococcus* sp. DC3A4 (NCIMB 42774).
3. A composition according to item 1 or item 2, wherein the composition comprises two or more probiotics.
4. A composition according to item 3, wherein the composition comprises two or more probiotics in combination with only one prebiotic material.
5. A composition according to item 4, wherein a first probiotic is taken from a group comprising specific facultative anaerobic commensal bacteria, and a second probiotic is taken from a group comprising specific strictly anaerobic commensal bacteria.
6. A composition according to any preceding item, wherein the prebiotic material is substantially indigestible in the gastrointestinal system of a chicken.
7. A composition according to any preceding item, wherein the prebiotic material is a polymeric saccharide.
8. A composition according to item 7, wherein the polymeric saccharide is an oligosaccharide.
9. A composition according to item 7 or item 8, wherein the polymeric saccharide is selected from one or more of fructo-oligosaccharide, isomaltooligosaccharide, mannanoligosaccharide, galactooligosaccharide, xylo-oligosaccharide, arabinoxylo-oligosaccharide, glucooligosaccharide, soyoligosaccharide, pectic oligosaccharide, and inulin.
10. A composition according to any preceding item, further comprising a nutrient food source.
11. A composition according to item 10, wherein the nutrient food source is a source of protein, starch, amino acids, fat, or a combination of any one or more thereof.
12. A composition according to any preceding item, wherein the composition is a starter feed or grower feed.
13. A composition according to item 12, wherein a starter feed comprises a prebiotic in an amount between 55% to 95% (w/w) solids concentration syrup.
14. A composition according to any of items 12 or 13, wherein a starter feed comprises a prebiotic added at a dose rate between 0.50% to 5.00% (w/w complete starter feed).
15. A composition according to item 12, wherein a grower feed comprises a prebiotic in an amount between 55% to 95% (w/w) solids concentration syrup.
16. A composition according to any of items 12 or 15, wherein a grower feed comprises a prebiotic added at a dose rate between 0.20% to 5.00% (w/w complete grower feed).
17. A composition according to any preceding item for use in the treatment of enteric bacterial disease in poultry.
18. A composition according to item 17, wherein the enteric bacterial disease is infection by one or more of the following: *Clostridium perfringens, Salmonella* spp, pathogenic and toxigenic *Escherichia coli* (EPEC and ETEC).
19. A method of producing a composition according to any of the preceding items.

## Claims

1. A composition comprising:
(i) a probiotic selected from one or more of the bacteria *Lactobacillus crispatus* str. DC21.1 (NCIMB 42771), *Lactobacillus johnsonii* DC22.2 (NCIMB 42772), *Lactobacillus reuteri* DC1B4 (NCIMB 42773), *Ruminococcus* sp. DC3A4 (NCIMB 42774), and *Collinsella tanakaei;* and
(ii) a prebiotic material.

2. A composition according to claim 1, wherein the composition comprises two or more probiotics, wherein the composition optionally comprises two or more probiotics in combination with only one prebiotic material.

3. A composition according to claim 2 wherein a first probiotic is taken from a group comprising specific facultative anaerobic commensal bacteria, and a second probiotic is taken from a group comprising specific strictly anaerobic commensal bacteria.

4. A composition according to any preceding claim, wherein the prebiotic material is substantially indigestible in the gastrointestinal system of a chicken.

5. A composition according to any preceding claim, wherein the prebiotic material is a polymeric saccharide, wherein the polymeric saccharide is optionally an oligosaccharide.

6. A composition according to claim 5, wherein the polymeric saccharide is selected from one or more of fructo-oligosaccharide, isomaltooligosaccharide, mannanoligosaccharide, galactooligosaccharide, xylo-oligosaccharide, arabinoxylo-oligosaccharide, glucooligosaccharide, soyoligosaccharide, pectic oligosaccharide, and inulin.

7. A composition according to any preceding claim, further comprising a nutrient food source, wherein the nutrient food source is optionally a source of protein, starch, amino acids, fat, or a combination of any one or more thereof.

8. A composition according to any preceding claim, wherein the composition is a starter feed or grower feed.

9. A composition according to 8, wherein the starter feed comprises a prebiotic in an amount between 55% to 95% (w/w) solids concentration syrup.

10. A composition according to any of claims 8 or 9, wherein the starter feed comprises a prebiotic added at a dose rate between 0.50% to 5.00% (w/w complete starter feed).

11. A composition according to claim 8, wherein the grower feed comprises a prebiotic in an amount between 55% to 95% (w/w) solids concentration syrup.

12. A composition according to any of claims 8 or 11, wherein the grower feed comprises a prebiotic added at a dose rate between 0.20% to 5.00% (w/w complete grower feed).

13. A composition according to any preceding claim for use in the treatment of enteric bacterial disease in poultry.

14. A composition according to claim 13, wherein the enteric bacterial disease is infection by one or more of the following: *Clostridium perfringens, Salmonella* spp, pathogenic and toxigenic *Escherichia coli* (EPEC and ETEC).

15. A method of producing a composition according to any of the preceding claims, the method comprising combining:
(i) a probiotic selected from one or more of the bacteria *Lactobacillus crispatus* str. DC21.1 (NCIMB 42771), *Lactobacillus johnsonii* DC22.2 (NCIMB 42772), *Lactobacillus reuteri* DC1B4 (NCIMB 42773), *Ruminococcus* sp. DC3A4 (NCIMB 42774), and *Collinsella tanakaei;* and
(ii) a prebiotic material.


Sequence Listing in EP20192478.4

<110>	Connerton, Ian; Connerton, Phillippa; Fish, Neville Marshall;
	Lafontaine, Geraldine; Richards, Phillip;	Dairy Crest Ltd

<120>	Composition for Treatment and/or Nutrition of Poultry

<140>	EP20192478.4
<141>	2017-06-30

<160>	22

<210>	1
<211>	19
<212>	DNA
<213>	Unknown

<220>
<221>	primer_bind
<222>	515F
<223>	Forward primer used to amplify the V4 region of the bacterial
16S rRNA
	gene from microbiota DNA isolated from the gastrointestinal
tract of
	chicken (Gallus gallus).

<300>
<301>	Caporaso, J.G., Lauber, C.L., Walters, W.A., Berg-Lyons, D.,
	Lozupone, C.A., Turnbaugh, P.J., Fierer, N. and Knight, R.
<302>	Global patterns of 16S rRNA diversity at a depth of millions of
	sequences per sample
<303>	PNAS
<304>	108
<305>	Supplement 1
<306>	4516-4522
<307>	2010-06-03

<400>	1

gtgccagcmg ccgcggtaa
19


<210>	2
<211>	20
<212>	DNA
<213>	Unknown

<220>
<221>	primer_bind
<222>	806R
<223>	Reverse primer used to amplify the V4 region of the bacterial
16S rRNA
	gene from microbiota DNA isolated from the gastrointestinal
tract of
	chicken (Gallus gallus).

<300>
<301>      Caporaso, J.G., Lauber, C.L., Walters, W.A., Berg-Lyons, D.,
           Lozupone, C.A., Turnbaugh, P.J., Fierer, N. and Knight, R.
<302>      Global patterns of 16S rRNA diversity at a depth of millions of
           sequences per sample
<303>      PNAS
<304>      108
<305>      Supplement 1
<306>      4516-4522
<307>      2010-06-03

<400>      2

ggactachvg ggtwtctaat
20



<210>      3
<211>      20
<212>      RNA
<213>      Gallus gallus

<220>
<221>      primer_bind
<222>      28F
<223>      Forward primer used to determine the mRNA level expression in
the

           chicken (Gallus gallus) of GAPDH, a housekeeping gene.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_204305.1   NCBI Nucleotide database

<400>      3

gacgtgcagc aggaacacta
20



<210>      4
<211>      20
<212>      RNA
<213>      Gallus gallus

<220>
<221>      primer_bind
<222>      370R
<223>      Reverse primer used to determine the mRNA level expression in
the

           chicken (Gallus gallus) of GAPDH, a housekeeping gene.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_204305.1   NCBI Nucleotide database

<400>      4

tctccatggt ggtgaagaca
20



<210>      5
<211>      20
<212>      RNA
<213>      Gallus gallus

<220>
<221>      primer_bind
<222>      320F
<223>      Forward primer used to determine the mRNA level expression in
the
           chicken (Gallus gallus) of IFN-γ, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_205149.1   NCBI Nucleotide database

<400>      5

tgagccagat tgtttcgatg
20



<210>      6
<211>      19
<212>      RNA
<213>      Gallus gallus

<220>
<221>      primer_bind
<222>      471R

<223>      Reverse primer used to determine the mRNA level expression in
the
          chicken (Gallus gallus) of IFN-γ, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
          chickens infected with avian H9N2 influenza virus
<303>      BMC Veterinary Research
<304>      42
<305>
<306>      64
<307>      2011-05-18
<308>      NM_205149.1   NCBI Nucleotide database

<400>      6

cttggccagg tccatgata
19



<210>      7
<211>      21
<212>      RNA
<213>      Gallus gallus

<220>
<221>      primer_bind
<222>      134F
<223>      Forward primer used to determine the mRNA level expression in
the
          chicken (Gallus gallus) of IFN-1β, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
          chickens infected with avian H9N2 influenza virus
<303>      BMC Veterinary Research
<304>      42
<305>
<306>      64
<307>      2011-05-18
<308>      NM_204524.1   NCBI Nucleotide database

<400>      7

ggattctgag cacaccacag t
21



<210>      8
<211>      22
<212>      RNA
<213>      Gallus gallus

<220>

<221>      primer_bind
<222>      405R
<223>      Reverse primer used to determine the mRNA level expression in
the
           chicken (Gallus gallus) of IFN-1β, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_204524.1   NCBI Nucleotide database

<400>      8

tctggttgat gtcgaagatg tc
22



<210>      9
<211>      20
<212>      RNA
<213>      Gallus gallus

<220>
<221>      primer_bind
<222>      99F
<223>      Forward primer used to determine the mRNA level expression in
the
           chicken (Gallus gallus) of IFN-4, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_001007079.1   NCBI Nucleotide database

<400>      9

ggagagcatc cggatagtga
20



<210>      10
<211>      20
<212>      RNA
<213>      Gallus gallus

<220>
<221>      primer_bind
<222>      284R
<223>      Reverse primer used to determine the mRNA level expression in
the
           chicken (Gallus gallus) of IFN-4, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_001007079.1   NCBI Nucleotide database

<400>      10

tgacgcatgttgaggaagag
20



<210>      11
<211>      20
<212>      RNA
<213>      Gallus gallus

<220>
<221>      primer_bind
<222>      249F
<223>      Forward primer used to determine the mRNA level expression in
the
           chicken (Gallus gallus) of IFN-10, a cytokine.

<300>
<301>      Nang, N.T., Lee, J.S., Song, B.M., Kang, Y.M., Kim, H.S. and
Seo, S.H.
<302>      Induction of inflammatory cytokines and toll-like receptors in
           chickens infected with avian H9N2 influenza virus
<303>      BMC Veterinary Research
<304>      42
<305>
<306>      64
<307>      2011-05-18
<308>      NM_001004414.2   NCBI Nucleotide database

<400>      11

gctgcgcttc tacacagatg
20



<210>      12
<211>      20

<212>	RNAxs
<213>	Gallus gallus

<220>
<221>	primer_bind
<222>	451R
<223>	Reverse primer used to determine the mRNA level expression in
the
	chicken (Gallus gallus) of IFN-10, a cytokine.

<300>
<301>	Nang, N.T., Lee, J.S., Song, B.M., Kang, Y.M., Kim, H.S. and
Seo, S.H.
<302>	Induction of inflammatory cytokines and toll-like receptors in
	chickens infected with avian H9N2 influenza virus
<303>	BMC Veterinary Research
<304>	42
<305>
<306>	64
<307>	2011-05-18
<308>	NM_001004414.2   NCBI Nucleotide database

<400>	12

tcccgttctc atccatcttc
20


<210>	13
<211>	15
<212>	RNA
<213>	Gallus gallus

<220>
<221>	primer_bind
<222>	391F
<223>	Forward primer used to determine the mRNA level expression in
the
	chicken (Gallus gallus) of IFN-6, a cytokine.

<300>
<301>	Kaiser, P., Underwood, G. and Davison, F
<302>	Differential Cytokine Responses following Marek's Disease Virus
	Infection of Chickens Differing in Resistance to Marek's
Disease
<303>	Journal of Virology
<304>	77
<305>	1
<306>	762-768
<307>	2003-01-01
<308>	NM_204628.1   NCBI Nucleotide database

<400>	13

gctcgccggc ttcga
15

<210>      14
<211>      22
<212>      RNA
<213>      Gallus gallus

<220>
<221>      primer_bind
<222>      461R
<223>      Reverse primer used to determine the mRNA level expression in
the
           chicken (Gallus gallus) of IFN-6, a cytokine.

<300>
<301>      Kaiser, P., Underwood, G. and Davison, F
<302>      Differential Cytokine Responses following Marek's Disease Virus
           Infection of Chickens Differing in Resistance to Marek's
Disease
<303>      Journal of Virology
<304>      77
<305>      1
<306>      762-768
<307>      2003-01-01
<308>      NM_204628.1   NCBI Nucleotide database

<400>      14

ggtaggtctg aaaggcgaac ag
22



<210>      15
<211>      22
<212>      RNA
<213>      Gallus gallus

<220>
<221>      primer_bind
<222>      323F
<223>      Forward primer used to determine the mRNA level expression in
the
           chicken (Gallus gallus) of IFN-17A, a cytokine.

<300>
<301>      Reid, W.D.K., Close, A.J., Humphrey, S., Chaloner, G.,
           Lacharme-Lora, L., Rothwell, L., Kaiser, P., Williams, N.J.,
           Humphrey, T.J., Wigley P. and Rushton, S.P.
<302>      Cytokine responses in birds challenged with the human food-
borne
           pathogen Campylobacter jejuni implies a Th17 response
<303>      Open Science
<304>      3
<305>
<306>      150541
<307>      2016-03-01
<308>      NM_204460.1   NCBI Nucleotide database

<400>      15

catgggatta caggatcgat ga
22

<210>	16
<211>	16
<212>	RNA
<213>	Gallus gallus

<220>
<221>	primer_bind
<222>	390R
<223>	Reverse primer used to determine the mRNA level expression in
the
	chicken (Gallus gallus) of IFN-17A, a cytokine.

<300>
<301>	Reid, W.D.K., Close, A.J., Humphrey, S., Chaloner, G.,
	Lacharme-Lora, L., Rothwell, L., Kaiser, P., Williams, N.J.,
	Humphrey, T.J., Wigley P. and Rushton, S.P.
<302>	Cytokine responses in birds challenged with the human food-
borne
	pathogen Campylobacter jejuni implies a Th17 response
<303>	Open Science
<304>	3
<305>
<306>	150541
<307>	2016-03-01
<308>	NM_204460.1   NCBI Nucleotide database

<400>	16

gcggcactgg gcatca
16


<210>	17
<211>	22
<212>	RNA
<213>	Gallus gallus

<220>
<221>	primer_bind
<222>	2742F
<223>	Forward primer used to determine the mRNA level expression in
the
	chicken (Gallus gallus) of IFN-17F, a cytokine.

<300>
<301>	Reid, W.D.K., Close, A.J., Humphrey, S., Chaloner, G.,
	Lacharme-Lora, L., Rothwell, L., Kaiser, P., Williams, N.J.,
	Humphrey, T.J., Wigley P. and Rushton, S.P.
<302>	Cytokine responses in birds challenged with the human food-
borne
	pathogen Campylobacter jejuni implies a Th17 response
<303>	Open Science
<304>	3
<305>

<306>      150541
<307>      2016-03-01
<308>      XM_426223.5   NCBI Nucleotide database

<400>      17

tgaccctgcc tctaggatga tc
22



<210>      18
<211>      20
<212>      RNA
<213>      Gallus gallus

<220>
<221>      primer_bind
<222>      2819R
<223>      Forward primer used to determine the mRNA level expression in
the
           chicken (Gallus gallus) of IFN-17F, a cytokine.

<300>
<301>      Reid, W.D.K., Close, A.J., Humphrey, S., Chaloner, G.,
           Lacharme-Lora, L., Rothwell, L., Kaiser, P., Williams, N.J.,
           Humphrey, T.J., Wigley P. and Rushton, S.P.
<302>      Cytokine responses in birds challenged with the human food-
borne
           pathogen Campylobacter jejuni implies a Th17 response
<303>      Open Science
<304>      3
<305>
<306>      150541
<307>      2016-03-01
<308>      XM_426223.5   NCBI Nucleotide database

<400>      18

gggtcctcat cgagcctgta
20



<210>      19
<211>      19
<212>      RNA
<213>      Gallus gallus

<220>
<221>      primer_bind
<222>      180F
<223>      Forward primer used to determine the mRNA level expression in
the
           chicken (Gallus gallus) of ChCXCLi1 (IL8L1), a cytokine.

<300>
<301>      Rasoli, M., Yeap, S.K., Tan, S.W., Roohani, K., Kristeen-Teo,
Y.W.,

Alitheen, N.B., Rahaman, Y.A., Aini, I., Bejo, M.H., Kaiser, P.
and
        Omar, A.R.
<302>   Differential modulation of immune response and cytokine
profiles in
        the bursae and spleen of chickens infected with very virulent
        infectious bursal disease virus
<303>   BMC Veterinary Research
<304>   11
<305>
<306>   75
<307>   2015-03-25
<308>   NM_205018.1   NCBI Nucleotide database

<400>   19

ccgatgccag tgcatagag
19



<210>   20
<211>   20
<212>   RNA
<213>   Gallus gallus

<220>
<221>   primer_bind
<222>   370R
<223>   Reverse primer used to determine the mRNA level expression in
the
        chicken (Gallus gallus) of ChCXCLi1 (IL8L1), a cytokine.

<300>
<301>   Rasoli, M., Yeap, S.K., Tan, S.W., Roohani, K., Kristeen-Teo,
Y.W.,
        Alitheen, N.B., Rahaman, Y.A., Aini, I., Bejo, M.H., Kaiser, P.
and
        Omar, A.R.
<302>   Differential modulation of immune response and cytokine
profiles in
        the bursae and spleen of chickens infected with very virulent
        infectious bursal disease virus
<303>   BMC Veterinary Research
<304>   11
<305>
<306>   75
<307>   2015-03-25
<308>   NM_205018.1   NCBI Nucleotide database

<400>   20

ccttgtccag aattgccttg
20



<210>   21
<211>   20
<212>   RNA

<213>      Gallus gallus

<220>
<221>      primer_bind
<222>      113F
<223>      Forward primer used to determine the mRNA level expression in
the
           chicken (Gallus gallus) of ChCXCLi2 (IL8L2), a cytokine.

<300>
<301>      Rasoli, M., Yeap, S.K., Tan, S.W., Roohani, K., Kristeen-Teo,
Y.W.,
           Alitheen, N.B., Rahaman, Y.A., Aini, I., Bejo, M.H., Kaiser, P.
and
           Omar, A.R.
<302>      Differential modulation of immune response and cytokine
profiles in
           the bursae and spleen of chickens infected with very virulent
           infectious bursal disease virus
<303>      BMC Veterinary Research
<304>      11
<305>
<306>      75
<307>      2015-03-25
<308>      NM_205498.1   NCBI Nucleotide database

<400>      21

cctggtttca gctgctctgt
20



<210>      22
<211>      20
<212>      RNA
<213>      Gallus gallus

<220>
<221>      primer_bind
<222>      240R
<223>      Reverse primer used to determine the mRNA level expression in
the
           chicken (Gallus gallus) of ChCXCLi2 (IL8L2), a cytokine.

<300>
<301>      Rasoli, M., Yeap, S.K., Tan, S.W., Roohani, K., Kristeen-Teo,
Y.W.,
           Alitheen, N.B., Rahaman, Y.A., Aini, I., Bejo, M.H., Kaiser, P.
and
           Omar, A.R.
<302>      Differential modulation of immune response and cytokine
profiles in
           the bursae and spleen of chickens infected with very virulent
           infectious bursal disease virus
<303>      BMC Veterinary Research
<304>      11
<305>
<306>      75

<307>      2015-03-25
<308>      NM_205498.1   NCBI Nucleotide database

<400>      22

gcgtcagctt cacatcttga
20